(19) Europäisches Patentamt
European Patent Office
Office européen des brevets

(11) Publication number: **0 235 823 B1**

(12) # EUROPEAN PATENT SPECIFICATION

(45) Date of publication of patent specification: **20.05.92**

(51) Int. Cl.5: **C07D 487/04**, A61K 31/40, //(C07D487/04,209:00,205:00)

(21) Application number: **87103189.4**

(22) Date of filing: **06.03.87**

The file contains technical information submitted after the application was filed and not included in this specification

(54) **Carbapenem antibiotics, a process for preparing them and pharmaceutical compositions containing them.**

(30) Priority: **06.03.86 US 837101**

(43) Date of publication of application: **09.09.87 Bulletin 87/37**

(45) Publication of the grant of the patent: **20.05.92 Bulletin 92/21**

(84) Designated Contracting States: **AT BE CH DE ES FR GB GR IT LI LU NL SE**

(56) References cited:
**EP-A- 0 168 707**
**GB-A- 2 122 196**

(73) Proprietor: **Bristol-Myers Squibb Company**
**345 Park Avenue**
**New York, N.Y. 10154(US)**

(72) Inventor: **Dextraze, Pierre**
**6600 Place de la Bataille**
**Laprairie, Ouebec J5R 3X8(CA)**

(74) Representative: **Kinzebach, Werner, Dr.**
**Patentanwälte Reitstötter, Kinzebach und Partner Sternwartstrasse 4 Postfach 86 06 49**
**W-8000 München 86(DE)**

EP 0 235 823 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.08/2.19/2.0)

## Description

BACKGROUND OF THE INVENTION

1. Field of the Invention

The present invention is directed to new carbapenem antibiotics in which the 2-substituent has the formula

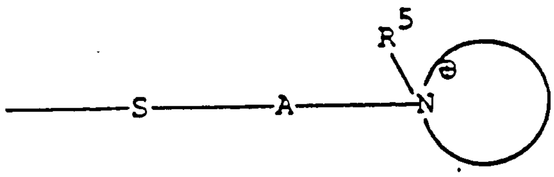

in which A

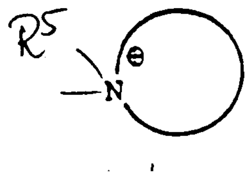

have the meanings given below.

2. Description of the Prior Art

A number of $\beta$-lactam derivatives containing the carbapenem nucleus

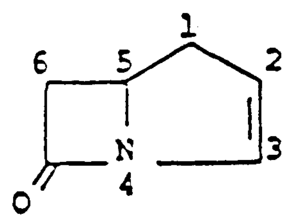

have been disclosed in the literature. These carbapenem derivatives have been reported to possess utility as antibacterial agents and/or $\beta$-lactamase inhibitors.

South African Patent Application 83/4472 discloses carbapenem derivatives of the general formula

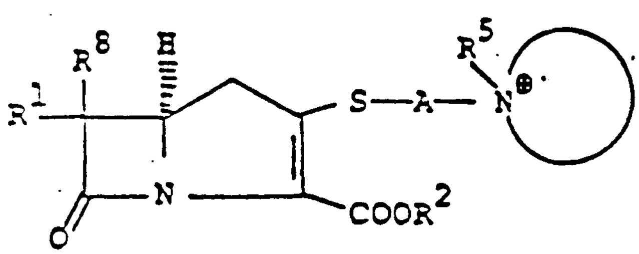

wherein $R^8$ is hydrogen and $R^1$ is selected from the group consisting of hydrogen; substituted and unsubstituted: alkyl, alkenyl and alkynyl, having from 1-10 carbon atoms; cycloalkyl and cycloalkylalkyl, having 3-6 carbon atoms in the cycloalkyl ring and 1-6 carbon atoms in the alkyl moieties; phenyl; aralkyl, aralkenyl and aralkynyl wherein the aryl moiety is phenyl and the aliphatic portion has 1-6 carbon atoms;

heteroaryl, heteroaralkyl, heterocyclyl and heterocyclylalkyl wherein the hetero atom or atoms in the above-named heterocyclic moieties are selected from the group consisting of 1-4 oxygen, nitrogen or sulfur atoms and the alkyl moieties associated with said heterocyclic moieties have 1-6 carbon atoms; wherein the substituent or substituents relative to the above-named radicals are independently selected from the group

consisting of $C_1$-$C_6$ alkyl optionally substituted by amino, halo, hydroxy or carboxyl halo

$-OR^3$

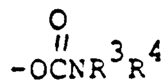

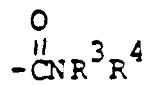

$-NR^3R^4-$

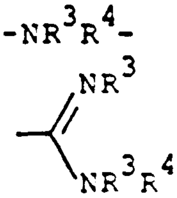

$-SO_2NR^3R^4$

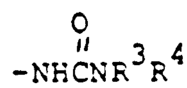

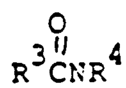

$-CO_2R^3$

$=O$

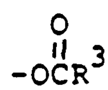

$-SR^3$

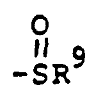

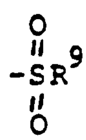

$-CN$

$-N_3$

$-OSO_3R^3$

$-OSO_2R^3$

$-NR^3SO_2R^4$

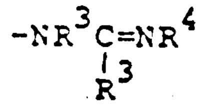

$-NR^3CO_2R^4$

$-NO_2$

wherein, relative to the above-named substituents, the groups $R^3$ and $R^4$ are independently selected from hydrogen; alkyl, alkenyl and alkynyl, having from 1-10 carbon atoms; cycloalkyl, cycloalkylalkyl and alkylcycloalkyl, having 3-6 carbon atoms in the cycloalkyl ring and 1-6 carbon atoms in the alkyl moieties; phenyl; aralkyl, aralkenyl and aralkynyl wherein the aryl moiety is phenyl and the aliphatic portion has 1-6 carbon atoms; and heteroaryl, heteroaralkyl, heterocyclyl and heterocyclylalkyl wherein the hetero atom or atoms in the above-named heterocyclic moieties are selected from the group consisting of 1-4 oxygen, nitrogen or sulfur atoms and the alkyl moieties associated with said heterocyclic moieties have 1-6 carbon atoms, or $R^3$ and $R^4$ taken together with the nitrogen to which at least one is attached may form a 5-or 6-membered nitrogen-containing heterocyclic ring; $R^9$ is as defined for $R^3$ except that it may not be hydrogen; or wherein $R^1$ and $R^8$ taken together represent $C_2$-$C_{10}$ alkylidene or $C_2$-$C_{10}$ alkylidene substituted by hydroxy; $R^5$ is selected from the group consisting of substituted and unsubstituted: alkyl, alkenyl and alkynyl, having from 1-10 carbon atoms; cycloalkyl and cycloalkylalkyl, having 3-6 carbon atoms in the cycloalkyl ring and 1-6 carbon atoms in the alkyl moieties; phenyl; aralkyl, aralkenyl and aralkynyl wherein the aryl moiety is phenyl and the aliphatic portion has 1-6 carbon atoms; heteroaryl, heteroaralkyl, heterocyclyl and heterocyclylalkyl wherein the hetero atom or atoms in the above-named heterocyclic moieties are selected from the group consisting of 1-4 oxygen, nitrogen or sulfur atoms and the alkyl moieties associated with said heterocyclic moieties have 1-6 carbon atoms; wherein the above-named $R^5$ radicals are optionally substituted by 1-3 substituents independently selected from: $C_1$-$C_6$ alkyl optionally substituted by amino, fluoro, chloro, carboxyl, hydroxy or carbamoyl; fluoro, chloro or bromo;

$-OR^3$ ;
$-OCO_2R^3$ ;
$-OCOR^3$ ;
$-OCONR^3R^4$ ;
$-OSO_2R^3$ ;
-oxo ;
$-NR^3R^4$ ;
$R^3CONR^4$- ;
$-NR^3CO_2R^4$ ;
$-NR^3CONR^3R^4$ ;
$-NR^3SO_2R^4$ ;
$-SR^3$ ;

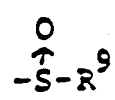 ;

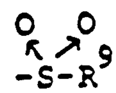 ;

$-SO_3R^3$ ;
$-CO_2R^3$ ;
$-CONR^3R^4$ ;
-CN; or
phenyl optionally substituted by 1-3 fluoro, chloro, bromo, $C_1$-$C_6$ alkyl, $-OR^3$, $-NR^3R^4$, $-SO_3R^3$, $-CO_2R^3$ or

-CONR$^3$R$^4$, wherein R$^3$, R$^4$ and R$^9$ in such R$^5$ substituents are as defined above; or R$^5$ may represent a divalent phenylene or $C_1$-$C_4$ alkylene group joined to the

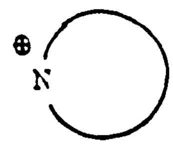

ring so as to form a bridged polycyclic group; A is cyclopentylene, cyclohexylene or $C_2$-$C_6$ alkylene optionally substituted by one or more $C_1$-$C_4$ alkyl groups; R$^2$ is hydrogen, an anionic charge or a conventional readily removable carboxyl protecting group, providing that when R$^2$ is hydrogen or a protecting group, there is also present a counter ion; and

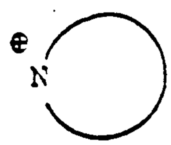

represents a substituted or unsubstituted mono-, bi- or polycyclic non-aromatic heterocyclic radical containing at least one nitrogen in the ring and attached to A through a ring nitrogen, thereby forming a quaternary ammonium group; and pharmaceutically acceptable salts thereof.

An alternate process for preparing the quaternized derivatives of South African Patent Application 83/4472 is disclosed in Belgian Patent 899,094.

The compounds disclosed in the above-cited references are all unsubstituted at the 1-position. Surprisingly, it has now been found that certain non-hydrogen 1-substituents impart a high degree of stability in the quaternized reference compounds.

## SUMMARY OF THE INVENTION

The present invention relates to carbapenem derivatives having the formula:

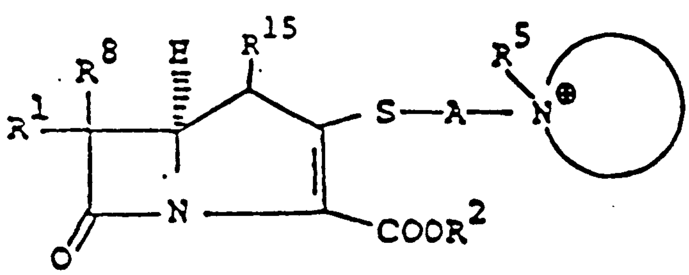

I

wherein

R$^8$ is hydrogen and R$^1$ is selected from the group consisting of hydrogen; substituted and unsubstituted alkyl having from 1 - 10 carbon atoms,
wherein the substituent is selected from the group consisting of
-OR$^3$

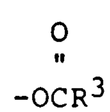

-$OSO_3R^3$
-$OSO_2R^3$
wherein $R^3$ is selected from hydrogen and alkyl having from 1 - 10 carbon atoms;
or
wherein $R^1$ and $R^8$ taken together represent $C_2$-$C_{10}$ alkylidene or $C_2$-$C_{10}$ alkylidene substituted by hydroxy;

$R^{15}$ is selected from the group consisting of alkyl having from 1 - 10 carbon atoms;

A is cyclopentylene, cyclohexylene or $C_2$-$C_6$ alkylene optionally substituted by one or more $C_1$-$C_4$ alkyl groups; $R^2$ is hydrogen, an anionic charge or a conventional readily removable carboxyl protecting group, providing that when $R^2$ is hydrogen or a protecting group, there is also present a counter ion; and

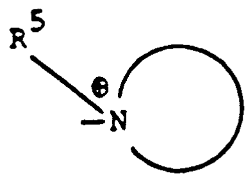

represents

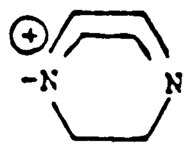

or

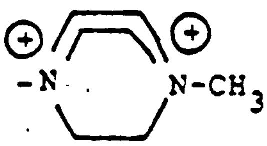

or a pharmaceutically acceptable salt thereof.

The compounds of formula I are potent antibacterial agents or intermediates useful in the preparation of such agents.

Also included in the invention are processes for preparing the novel carbapenem derivatives described above and pharmaceutical compositions containing the biologically active carbapenem derivatives in combination with pharmaceutically acceptable carriers or diluents.

## DETAILED DESCRIPTION

The novel compounds of general formula I above contain the carbapenem nucleus

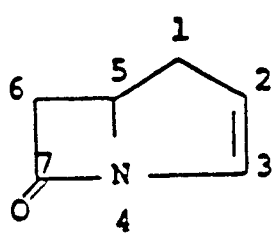

and may thus be named as 1-carba-2-penem-3-carboxylic acid derivatives. Alternatively, the compounds may be considered to have the basic structure

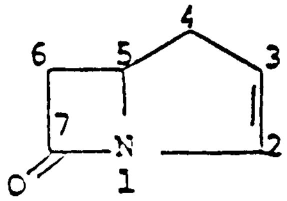

and named as 7-oxo-1-azabicyclo (3.2.0)hept-2-ene-2-carboxylic acid derivatives. While the present invention includes compounds wherein the relative stereochemistry of the 5,6-protons is cis as well as trans, the preferred compounds have the 5R,6S (trans) stereochemistry as in the case of thienamycin.

The compounds of formula I may be unsubstituted in the 6-position or substituted by substituent groups previously disclosed for other carbapenen derivatives. More specifically, $R^8$ may be hydrogen and $R^1$ may be hydrogen or a non-hydrogen substituent disclosed, for example, in European Patent Application 38,869 (see definition of $R_6$). Alternatively, $R^8$ and $R^1$ taken together may be $C_2$-$C_{10}$ alkylidene or $C_2$-$C_{10}$ alkylidene substituted, for example, by hydroxy.

The compounds of Formula I are substituted at the 1-position by a $C_1$-$C_{10}$ -alkyl group. Preferred non-hydrogen $R^{15}$ substituents include $C_1$-$C_6$ alkyl, most preferably methyl. The non-hydrogen $R^{15}$ substituent may be in either the $\alpha$- or $\beta$-configuration, and it is intended that the present invention include the individual $\alpha$- and $\beta$-isomers, as well as mixtures thereof. The most preferred 1-substituted compounds are those having the $\beta$-configuration, especially those having the $\beta$-methyl substituent.

To elaborate on the definitions for $R^1$, and $R^{15}$:
The aliphatic "alkyl" groups may be straight or branched chain having 1-10 carbon atoms; preferred are 1-6, most preferably 1-4, carbon groups .

The term "conventional readily removable carboxyl protecting group" refers to a known ester group which has been employed to block a carboxyl group during the chemical reaction steps described below and which can be removed, if desired, by methods which do not result in any appreciable destruction of the remaining portion of the molecule, e.g. by chemical or enzymatic hydrolysis, treatment with chemical reducing agents under mild conditions, irradiation with ultraviolet light or catalytic hydrogenation. Examples of such ester protecting groups include benzhydryl, ally), p-nitrobenzyl, 2-naphthylmethyl, benzyl, trichloroethyl, silyl such as trimethylsilyl, phenacyl, p-methoxybenzyl, acetonyl, o-nitrobenzyl, 4-pyridylmethyl and $C_1$-$C_6$ alkyl such as methyl, ethyl or t-butyl. Included within such protecting groups are those which are hydrolyzed under physiological conditions such as pivaloyloxymethyl, acetoxymethyl, phthalidyl, indanyl and methoxymethyl. Particularly advantageous carboxyl protecting groups are p-nitrobenzyl which may be readily removed by catalytic hydrogenolysis and allyl which can be removed by $Pd(PØ_3)_4$-catalyzed reaction.

The pharmaceutically acceptable salts referred to above include the nontoxic acid addition salts, e.g. salts with mineral acids such as hydrochloric, hydrobromic, hydroiodic, phosphoric, sulfuric, etc. and salts with organic acids such as maleic, acetic, citric, succinic, benzoic, tartaric, fumaric, mandelic, ascorbic, lactic, gluconic and malic. Compounds of formula I in the form of acid addition salts may be written as

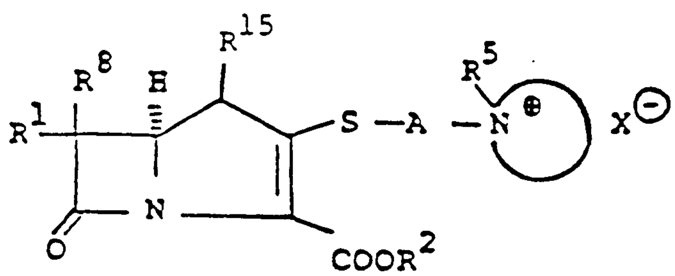

$R^2$ = H or protecting group

where $X^{\ominus}$ represent the acid anion. The counter anion $X^{\ominus}$ may be selected so as to provide pharmaceutically acceptable salts for therapeutic administration but, in the case of intermediate compounds of formula I, $X^{\ominus}$ may also be a toxic anion. In such a case the ion can be subsequently removed or substituted by a pharmaceutically acceptable anion to form an active end product for therapeutic use. When acidic groups are present in the $R^1$ radical, the present invention may also include suitable base or acid salts of these functional groups, e.g. metal salts (e.g. sodium, potassium, calcium and aluminum) , the ammonium salt and salts with nontoxic amines (e.g. trialkylamines, procaine, dibenzylamine, 1-ephenamine, N-benzyl-$\beta$-phenethylamine, N,N'-dibenzyl-ethylenediamine, etc.).

Compounds of formula I wherein $R^2$ is hydrogen, an anionic charge or a physiologically hydrolyzable ester group together with pharmaceutically acceptable salts thereof are useful as antibacterial agents. The remaining compounds of formula I are valuable intermediates which can be converted into the above-mentioned biologically active compounds.

A preferred embodiment of the present invention comprises compounds of formula I wherein $R^8$ is hydrogen and $R^1$ is hydrogen, $CH_3CH_2$-

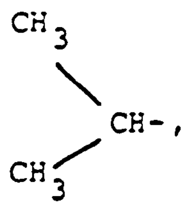

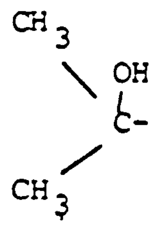

or

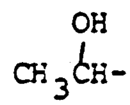

.

Among this subclass, the preferred compounds are those in which $R^1$ is

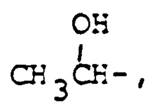

most preferably compounds having the absolute configuration 5R, 6S, 8R.

Another preferred embodiment comprises compounds of formula I in which $R^1$ and $R^8$ taken together form an alkylidene radical of the formula

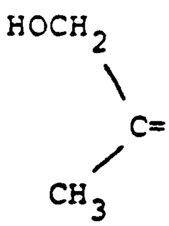

.

The alkylene A substituent is preferably straight or branched chain alkylene of from 2 to 6 carbon atoms. A cycloalkylene A substituent is preferably cyclopentylene of the formula

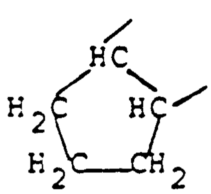

or cyclohexylene of the formula

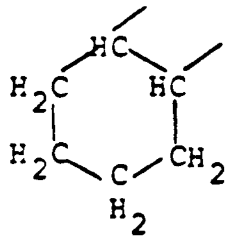

A preferred embodiment comprises those compounds in which A is

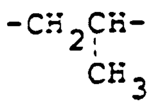 , 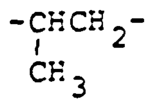 , 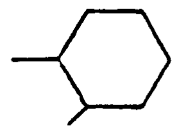

or $-(CH_2)_n-$ in which n is 2, 3 or 4 and a particularly preferred embodiment comprises those compounds where A is $-CH_2CH_2-$, $-CH_2CH_2CH_2-$

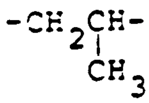 , 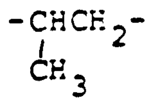 or 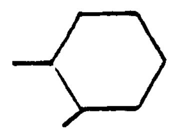 .

Most preferably A is $-CH_2-CH_2-$. Most preferably A is $-CH_2-CH_2-$.

The most preferred embodiments of the present invention comprise the compounds of the formula

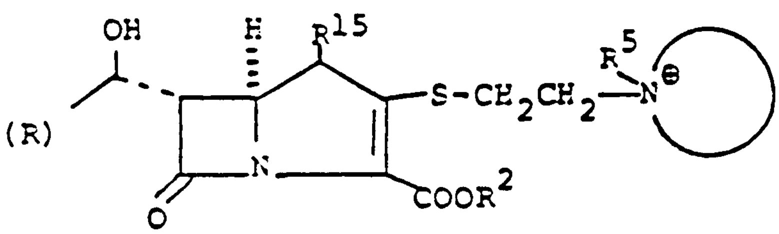

wherein $R^{15}$ is $\beta$-methyl and

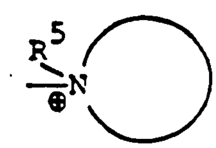

represents

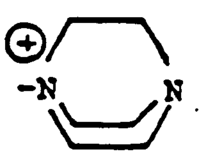 or 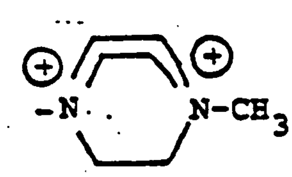

and $R^2$ is hydrogen, an anionic charge or a conventional readily removable carboxyl protecting group, providing that when $R^2$ is hydrogen or a protecting group, there is also present a counter ion, and pharmaceutically acceptable acid addition salts thereof.

It will be appreciated that certain products within the scope of formula I may be formed as optical isomers as well as epimeric mixtures thereof. It is intended that the present invention include within its scope all such optical isomers and epimeric mixtures. For example, when the 6-substituent is hydroxyethyl, such substituent may be in either the R or S configuration and the resulting isomers as well as epimeric mixtures thereof are encompassed by the present invention.

The carbapenem derivatives of general formula I are prepared from starting material of the formula

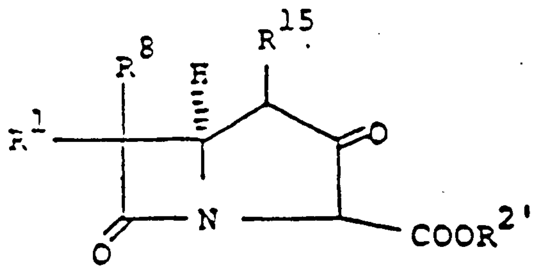

III

wherein $R^1$, $R^{15}$, and $R^8$ are defined above and wherein $R^{2'}$ represents a conventional readily removable carboxyl protecting group. Compounds of formula III have been disclosed, for example, in European Patent Application 38,869 (compound 7) and in European Patent Application 54,917, and may be prepared by the general methods described therein.

One process for preparing compounds I from starting materials III may be summerized by the following reaction scheme:

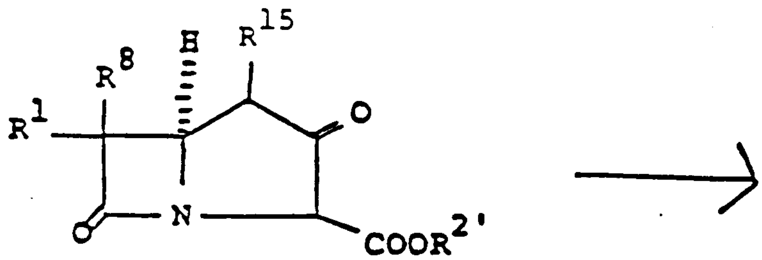

III

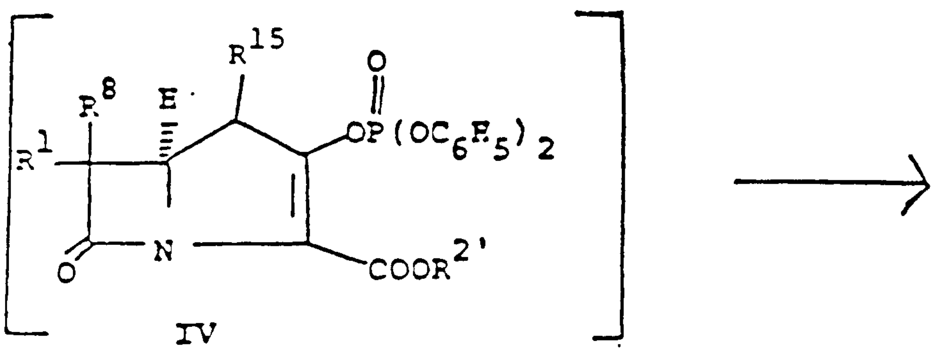

IV

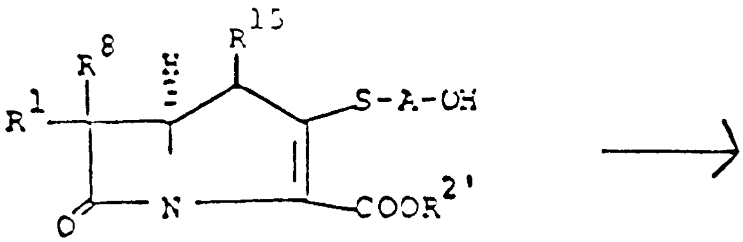

V

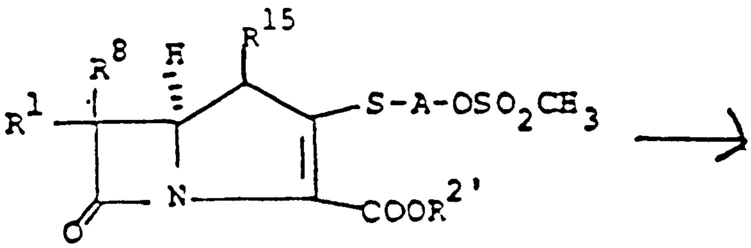

VI

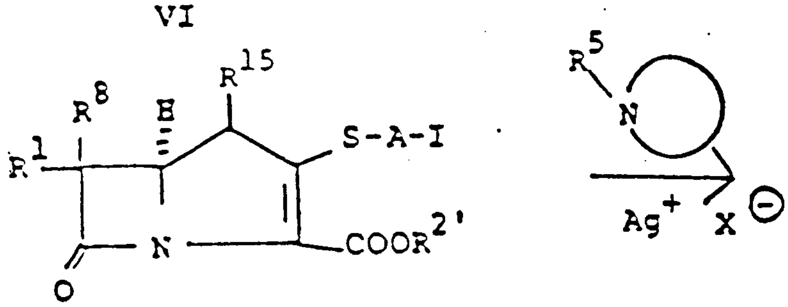


II

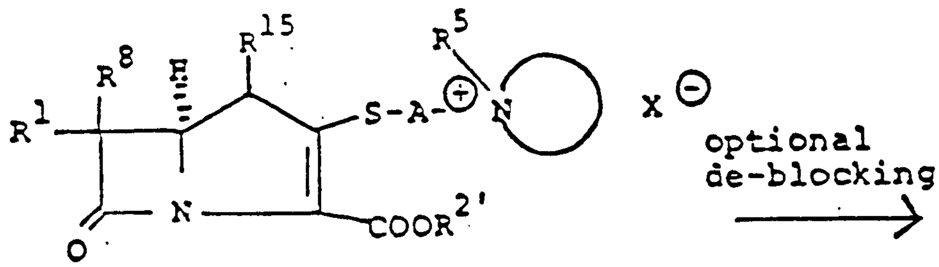


I'

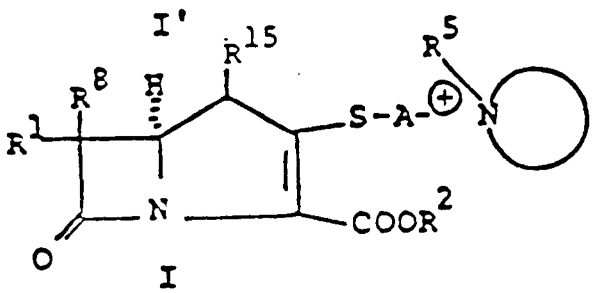

I

To elaborate on the above process, starting material III is reacted in the inert organic solvent such as methylene chloride, acetonitrile or dimethylformamide with about an equimolar amount of diphenyl chlorophosphate in the presence of a base such as diisopropylethylamine, triethylamine, 4-dimethylaminopyridine or the like to give intermediate IV. The acylation to establish the diphenyl-

phosphoryloxy leaving group at the 2-position of intermediate III is advantageously carried out at a temperature of from about -20° to -40° C, most preferably at about 0° C. Intermediate IV may be isolated if desired, but is conveniently used for the next step without isolation or purification.

Intermediate IV is next converted to intermediate V by a conventional displacement reaction. Thus, intermediate IV may be reacted with approximately an equimolar amount of a mercaptan reagent of the formula

HS-A-OH

wherein A represents cyclopentylene, cyclohexylene or $C_2$-$C_6$ alkylene optionally substituted by one or more $C_1$-$C_4$ alkyl groups in an inept organic solvent such as dioxane, dimethylformamide, dimethylsulfoxide or acetonitrile and in the presence of a base such as diisopropylethylamine, triethylamine, sodium hydrogen carbonate, potassium carbonate or 4-dimethylaminopyridine. The temperature for the displacement is not critical, but an advantageous temperature range is frog about -40° C to 25° C. Most conveniently, the reaction is carried out with cooling, e.g. at about 0° C.

Intermediate V is then acylated with methanesulfonyl chloride or a functional acylating equivalent thereof such as methanesulfonic acid anhydride in an inert organic solvent and in the presence of base to provide the methanesulfonyloxy leaving group of intermediate VI. The acylation is carried out in an inert organic solvent such as tetrahydrofuran, methylene chloride, acetonitrile or dimethylformamide and in the presence of a suitable base such as diisopropylethylamine, triethylamine, 4-dimethylaminopyridine, and the like. The reaction may be carried out over a wide temperature range, e.g. -40° C to +40° C, but is most advantageously conducted with cooling, e.g. at about -30° C to -40° C.

Intermediate VI is next subjected to a displacement reaction so as to provide in intermediate II the iodo leaving group. This particular group has been found to greatly facilitate preparation of the carbapenem end-products of formula I.

The displacement of the methanesulfonyloxy leaving group is carried out by reacting intermediate VI with a source of iodide ions in an inert organic solvent such as acetone, dimethyl-formamide or dimethylsulfoxide. Any compound which ionizes in the solvent employed to provide iodide ions may be used, e.g. an alkali metal iodide such as NaI or KI. The temperature for the displacement is not critical, but temperatures of room temperature or above are most advantageous for achieving completion of the reaction in a reasonable the period. The source of iodide ions is employed in an amount so as to provide approximately an equivalent or excess of iodide ion relative to intermediate VI.

Preparation of the desired carbapenem derivatives of formula I is carried out by a nucleophilic displacement of the iodo leaving group of intermediate II by the desired nitrogen-containing heterocyclic nucleophile

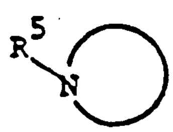 .

Intermediate II is reacted with at least an equivalent, preferably an excess, of the desired amine reagent in an inert organic solvent and in the presence of silver ion. Suitable inert organic solvents include, for example, tetrahydrofuran, dioxane, methylene chloride, diglyme, dimethoxyethane, and the like. Any silver compound which substantially ionizes in the solvent to give silver ions and an inert anion may be used as the source of silver ion, e.g. $AgClO_4$. Generally, we prefer to use approximately an equivalent amount (relative to intermediate II) of silver ion to facilitate the displacement. The reaction may be carried out over a wide temperature range, e.g. from about -25° to about +25° C, but is most preferably conducted at around 0° C. Intermediate I' will have a counter anion (e.g. derived from the silver salt used) associated with it which may at this stage be substituted by a different counter anion, e.g. one which is pharmaceutically acceptable, by conventional procedures. Alternatively, the counter ion may be subsequently removed during the de-blocking step.

The de-blocking step to remove the carboxyl protecting group $R^{2'}$ of intermediate I' is accomplished by conventional procedures such as solvolysis, chemical reduction or hydrogenation. Where a protecting group such as p-nitrobenzyl, benzyl, benzhydryl or 2-naphthylmethyl is used which can be removed by catalytic hydrogenation, intermediate I' in a suitable solvent such as dioxanewater-ethanol, tetrahydrofuran-diethylether-buffer, tetrahydrofuranaqueous dipotassium hydrogen phosphate-isopropanol or the like may be

treated under a hydrogen pressure of from 1 to 4 atmospheres in the presence of a hydrogenation catalyst such as palladium on charcoal, palladium hydroxide, platinum oxide or the like at a temperature of from 0 to 50 °C for from about 0.24 to 4 hours. When $R^{2'}$ is a group such as o-nitrobenzyl, photolysis may also be used for deblocking. Protecting groups such as 2,2,2-trichloroethyl may be removed by mild zinc reduction. The allyl protecting group may be removed with a catalyst comprising a mixture of a palladium compound and triphenyl phosphine in an aprotic solvent such as tetrahydrofuran, diethyl ether or methylene chloride. Similarly, other conventional carboxyl protecting groups may be removed by methods known to those skilled in the art. Finally, as mentioned above, compounds of formula I' where $R^{2'}$ is a physiologically hydrolyzable ester such as acetoxymethyl, phthalidyl, indanyl, pivaloyloxymethyl, methoxymethyl, etc. may be administered directly to the host without de-blocking since such esters are hydrolyzed in vivo under physiological conditions.

Preparation of certain compounds of general formula I may involve a further reaction step just prior to or following the de-blocking reaction. When the heterocyclic nitrogen-containing substituent has two quaternary nitrogen groups, e.g. as in the substituent

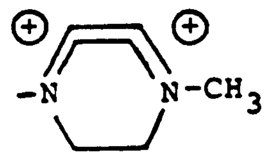

,

the product of the quaternization reaction may be subjected to a further quaternization reaction to obtain the di quaternary end-product.

An alternate process which can be used to prepare compounds of formula I comprises reacting an intermediate of the formula

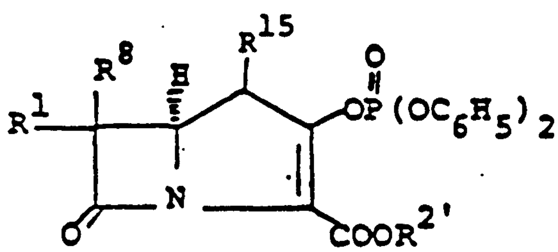

IV

wherein $R^1$ , $R^{15}$ and $R^8$ are us defined for the compounds of Formula I and $R^{2'}$ is a conventional readily removable carboxyl protecting group with a thiol compound of the formula

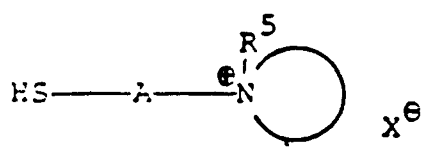

VII

wherein A and

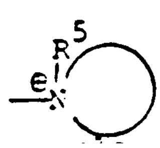

are as defined above in connection with the compounds of Formula I and $X^{\ominus}$ is a counter anion in an inert solvent and in the presence of base to produce the carbapenem product of the formula

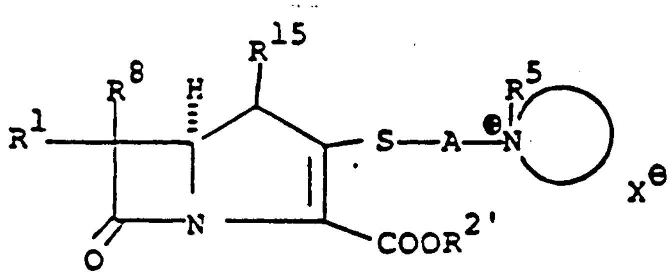

I'

wherein $R^1$, $R^8$, $R^{2'}$, $R^{15}$, A,

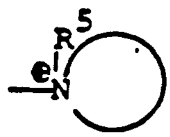

and $X^{\ominus}$ are as defined above and, if desired, removing the carboxyl protecting group $R^{2'}$ to give the corresponding de-blocked compound of Formula I, or a pharmaceutically acceptable salt thereof.

The above alternative process utilizes intermediate IV which may be prepared as described above for the general synthetic process. Intermediate IV is generally prepared in situ from intermediate III and used without isolation or purification.

To produce di quaternary end-products by this process, the intermediate IV may be reacted with the desired mono quaternary thiol compound VII and the so-produced carbapenem product then subjected to a further quaternization step as described above to give the di quaternary end-product. Alternatively, intermediate IV may be reacted directly with the di quaternary thiol VII to give the desired end-product.

In the alternative process, carbapenem intermediate IV is reacted with a quaternary amine thiol of the formula

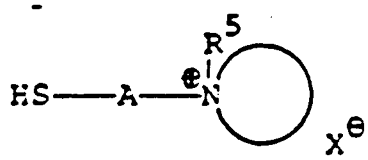

VII

wherein

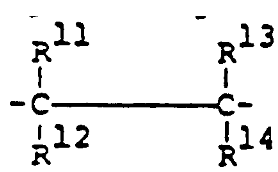

$X^{\ominus}$ is a counter anion associated with a strong acid such as $Cl^-$, $Br^-$, $CH_3SO_3^-$ $CF_3SO_3^-$ or

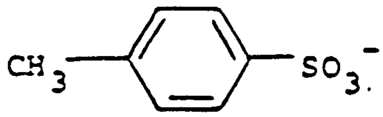 and 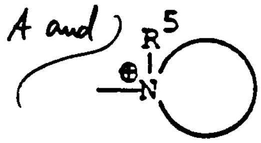

are as defined above. The reaction is carried out in an inert solvent such as acetonitrile, acetonitrile-$H_2O$, acetonitrile-dimethylformamide tetrahydrofuran, tetrahydrofuran-$H_2O$ or acetone in the presence of base The nature of the base is not critical. Best results, however, have been obtained when a non-nucleophilic tertiary amine base such as diisopropylethylamine, 1,8-diazabicyclo[5.4.0]undec-7-ene, 1,5-diazabicyclo[4.3.0]non-5-ene or a tri($C_1$-$C_4$)alkylamine such as triethylamine, tributylamine or tripropylamine is employed. Reaction of intermediate IV with thiol VII may be carried out over a wide temperature range, e.g. -15°C up to room temperature, but is preferably done at a temperature in the range of from about -15°C to +15°C, most preferably at around 0°C.

The carbapenem product produced by reaction of the quaternary amine thiol VII with intermediate IV will have a counter anion associated with it (i.e. $(C_6H_5O)_2PO_2^{\ominus}$, $Cl^{\ominus}$ or the anion associated with the quaternary thiol) which may at this stage be substituted by a different counter anion, e.g. one which is more pharmaceutically acceptable, by conventional procedures. Alternatively, the counter anion may be removed during the subsequent de-blocking step. Where the quaternized carbapenem compound and counter anion form an insoluble product, the product may crystallize out as it is formed and be collected pure by filtration.

Following formation of the desired carbapenem product according to the above-described reaction step, the carboxyl protecting group $R^{2'}$ of compound I' may be optionally removed by conventional procedures as described above in connection with the general synthetic process.

The thiol intermediates of Formula VII may be prepared, for example, by reacting a sulfide of the formula

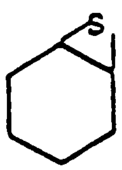 , 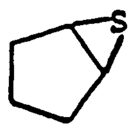 or 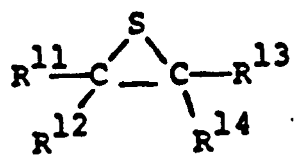 .

VIIIa VIIIb VIIIc

wherein $R^{11}$, $R^{12}$ $R^{13}$ and $R^{14}$ are each independently hydrogen or $C_1$-$C_4$ alkyl with a heterocyclic amine (as defined above) of the formula

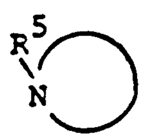

and a strong acid. The reaction may be carried out in the presence or absence of an inert organic solvent which is preferably a non-polar organic solvent such as methylene chloride, benzene, xylene, toluene or the like. Where the amine and sulfide reagents are liquids or where a solid amine is soluble in a liquid sulfide reagent, it is preferred to carry out the reaction without use of an additional solvent.

The particular strong acid used in the reaction is not critical and may be, for example, such strong inorganic or organic acids as hydrochloric, hydrobromic, methanesulfonic, p-toluenesulfonic, trifluoromethanesulfonic, etc.

Formation of the quaternary amine thiol intermediate VII may be carried out at a temperature in the range of from about -20 ° C to about 100 ° C. Preferred temperatures are generally in the range of about 50-70 ° C.

The sulfide reagent, aromatic amine and acid are preferably employed so that the sulfide and acid are used in approximately equimolar amounts with the amine being used in excess, e.g. two to three moles of amine per mole of sulfide or acid.

Alternatively the quaternary thiol intermediate VII may be prepared by reacting a protected thiol of the formula

PS-A-Halogen

where P is a conventional thiol protecting group, e.g. acetyl, halogen is a halo atom, preferably Br, I or Cl and most preferably Br, and A is as defined above in an inert solvent with the appropriate heterocyclic amine. This protected quaternary thiol may then be de-protected by conventional means to provide the quaternary thiol intermediate VII.

The quaternary amine thiol intermediate will have a counter anion associated with it which will be determined by the particular acid employed. It is, of course, possible to substitute at this point a different counter anion by conventional procedures for use in the subsequent reaction with carbapenem intermediate IV.

It will be understood that where the $R^1$, $R^8$ and/or $R^{15}$ substituent or the heterocyclic nucleophile attached to substituent A contain a functional group which might interfere with the intended course of reaction, such group may be protected by a conventional blocking group and then subsequently de-blocked to regenerate the desired functional group. Suitable blocking groups and procedures for introducing and removing such groups are well known to those skilled in the art.

In the case of certain compounds of formula I having a cycloalkylene or branched alkylene A substituent, one or more additional assymmetric carbon atoms may be created which result in formation of diastereoisomers. The present invention includes mixtures of such diastereoisomers as well as the individual purified diastereoisomers.

As in the case of other $\beta$-lactam antibiotics, compounds of general formula I may be converted by known procedure to pharmaceutically acceptable salts which, for purposes of the present invention, are substantially equivalent to the non-salted compounds. Thus, for example, one may dissolve a compound of formula I wherein $R^2$ is an anionic charge in a suitable inert solvent and then add an equivalent of a pharmaceutically acceptable acid. The desired acid addition salt may be recovered by conventional procedures, e,g. solvent precipitation, lyophilization, etc. Where other basic or acidic functional groups are present in the compound of formula I, pharmaceutically acceptable base addition salts and acid addition salts may be similarly prepared by known methods.

A compound of formula I where $R^2$ is hydrogen or an anionic charge, or a pharmaceutically acceptable salt thereof may also be converted by conventional procedures to a corresponding compound where $R^2$ is a physiologically hydrolyzable ester group, or a compound of formula I wherein $R^2$ is a conventional carboxyl protecting group may be converted to the corresponding compound where $R^2$ is hydrogen, an anionic charge or a physiologically hydrolyzable ester group, or a pharmaceutically acceptable salt thereof.

The novel carbapenem derivatives of general formula I wherein $R^2$ is hydrogen, an anionic charge or a physiologically hydrolyzable carboxyl protecting group, or the pharmaceutically acceptable salts thereof, are potent antibiotics active against various gram-positive and gram-negative bacteria and they may be used, for example, as animal feed additives for promotion of growth as preservatives in food, as bactericides in industrial applications, for example in waterbased paint and in the white water of paper mills to inhibit the growth of harmful bacteria and as disinfectants for destroying or inhibiting the growth of harmful bacteria on medical and dental equipment. They are especially useful, however, in the treatment of infectious disease in humans and other animals caused by gram-positive or gram-negative bacteria.

The pharmaceutically active compounds of this invention may be used alone or formulated as pharmaceutical compositions comprising, in addition to the active carbapenem ingredient, a pharmaceutically acceptable carrier or diluent. The compounds may be administered by a variety of means; those of principal interest include: orally, topically or parenterally (intravenous or intramuscular injection). The pharmaceutical compositions may be in solid from such as capsules, tablets, powders, etc. or in liquid form such as solutions, suspensions or emulsions. Compositions for injection, the preferred route of delivery, may be prepared in unit dose form in ampules or in multidose containers and may contain formulatory agents such as suspending, stabilizing and dispersing agents. The compositions may be in ready to use form or in powder form for reconstitution at the time of delivery with a suitable vehicle such as sterile water.

The dosage to be administered depends to a large extent on the particular compound being used, the particular composition formulated, the route of administration, the nature and condition of the host and the particular situs and organism being treated. Selection of the particular preferred dosage and route of application, then, is left to the discretion of the therapist. In general, however, the compounds may be administered parenterally or orally to mammalian hosts in an amount of from about 5 to 200 mg/kg/day. Administration is generally carried out in divided doses, e.g. three to four times a day.

To illustrate the potent broad-spectrum antibacterial activity of the carbapenems of the present invention, both in vitro and in vivo, biological data is provided below relating to the present preferred carbapenem compounds of the present invention.

In Vitro Activity

Samples of the carbapenem compounds of Examples 1 and 2 after solution in water and dilution with Nutrient Broth were found to exhibit the following Minimum Inhibitory Concentrations (M.I.C.) in mcg/ml versus the indicated microorganisms as determined by overnight incubation at 37 deg. C by tube dilution.

N-formimidoyl thienamycin was included as a comparison compound.

In Vitro Antibacterial Activity of Carbapenem Derivatives of Examples 1 and 2

| Organism | | MIC (mcg/ml) | | |
| --- | --- | --- | --- | --- |
| | | Compound of Ex. 1 | Compound of Ex. 2 | N-Formimidoyl Thienamycin |
| S.pneumoniae | A9585 | 0.03 | 0.016 | 0.002 |
| S.pyogenes | A9604 | 0.03 | 0.016 | 0.002 |
| S.faecalis | A20688 | 2 | 1 | 0.25 |
| S.aureus | A9537 | 0.5 | 0.016 | 0.004 |
| S.aureus + 50% serum | A9537S | 0.13 | - | 0.016 |

| Organism | | MIC (mcg/ml) Compound of Ex. 1 | Compound of Ex. 2 | N-Formimidoyl Thienamycin |
|---|---|---|---|---|
| S. aureus (penicillin resistant) | A9606 | 0.5 | 0.03 | 0.008 |
| S. aureus (methicillin resistant) | A20699 | 32 | 63 | 32 |
| E. coli | A15119 | 0.06 | 0.06 | 0.016 |
| E. coli | A20341-1 | 0.13 | 0.13 | 0.03 |
| K. pneumoniae | A9664 | 0.13 | 0.06 | 0.03 |
| K. pneumoniae | A20468 | 0.06 | 0.25 | 0.06 |
| E. cloacae | A9659 | 0.5 | 0.13 | 0.13 |
| E. cloacae | A9656 | 0.25 | 0.13 | 0.03 |
| P. mirabilis | A9900 | 0.13 | 0.13 | 0.03 |
| P. vulgaris | A21559 | 0.5 | 0.03 | 0.03 |
| M. morganii | A15153 | 0.25 | 0.13 | 0.06 |
| P. rettgeri | A22424 | 0.5 | 1 | 0.13 |
| S. marcescens | A20019 | 0.25 | 0.13 | 0.03 |
| P. aeruginosa | A9843a | 0.5 | 0.5 | 1 |
| P. aeruginosa | A21213 | 0.5 | 0.13 | 0.06 |
| P. aeruginosa | A21628 | - | 4 | 4 |

In Vivo Activity

The in vivo therapeutic efficacy of the compounds of Examples 1 and 2 and N-formimidoyl thienamycin after intramuscular administration to mice experimentally infected with P.aeruginosa A9843a is shown in the following table. The $PD_{50}$ (dose in mg/kg required to give protection to 50% of the infected mice) is indicated. The animals were treated 0 and 2 hours after infection.

| Protective Effect in the Intramuscular Treatment of Infected Mice | | | | |
|---|---|---|---|---|
| Organism | Challenge (No. of Organisms | Comp. of Ex. 1 | Comp. of Ex. 2 | N-Formimidoyl Thienamycin |
| P.aeruginosa A9843a | $6 \times 10^4$ | 1.6 | | |
| | $2 \times 10^4$ | | 2.0 | 0.39 |

Example 1

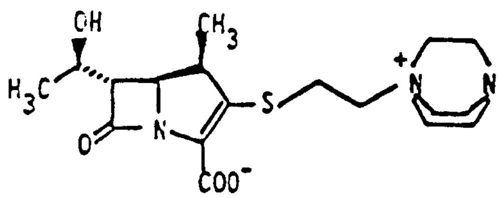

(5R,6S) 3-[2-(1,4-diazabicyclo[2.2.2.]octanium-1-yl)ethylthio]-6-(1R-hydroxyethyl)-4R-methyl-7-oxo-1-azabicyclo[3.2.0]hept-2-ene-2-carboxylate .

1-(2-acetylthioethyl)-1,4-diazabicyclo[2.2.2]octanium bromide

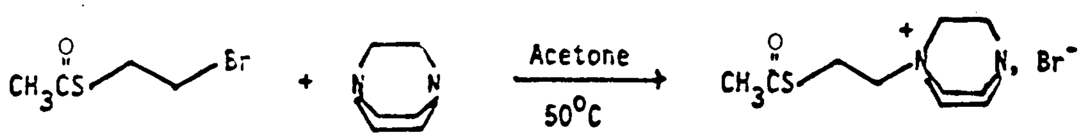


A mixture of 2-bromoethylthiol acetate (4.0 g, 21.8 mmol) and 1,4-diazabicyclo[2.2.2]octane (2.85 g, 25.4 mmol) in dry acetone (8 mL) was stirred at 50 °C under nitrogen atmosphere for 65 h. After cooling to 23 °C, the liquid phase was decanted and the gum was triturated in ether (3x10 mL). On standing, the gum crystallized slowly to give after drying under high vacuum 5.8 g (90%) of white crystals, mp 145-55 °C. Trituration in acetone afforded an analytical sample, mp 154-160 °C; ir (KBr) $V_{max}$ : 1688 (C=O of thioacetate) $cm^{-1}$; $^1$Hmr ($D_2O$) $\delta$: 2.45 (s, $CH_3$), 2.7-3.8 (m, $8CH_2$); Anal. calcd for $C_{10}H_{19}N_2OSBr$: C 40.68, H 6.49, N 9.49, S 10.86; found: C 40.30, H 6.56, N 9.52, S 10.66

1(2-mercaptoethyl)-1,4-diazabicyclo[2.2.2]octanium bromide, hydrochloride

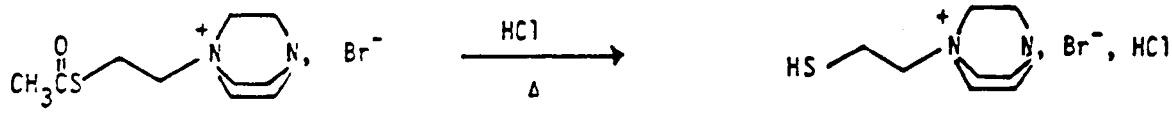


A solution of 1-(2-acetylthioethyl)-1,4-diazabicyclo[2.2.2]octanium bromide (5.6 g, 18.9 mmol) in 6N hydrochloric acid (21 mL) was stirred at 80 °C under nitrogen atmosphere for 1 h, cooled to 23 °C and concentrated to dryness. After drying under high vacuum overnite, the white solid was kept over $P_2O_5$ - (56 °C) under high vacuum until constant weight, 5.2 g, 95%; ir (KBr) $\nu_{max}$ : 2700-2000 ($N^+$-H) $cm^{-1}$; $^1$Hmr (DMSO, d-6) δ: 2.7-3.3 (m, $SCH_2$), 3.3-4.5 (m, $7N^+$-$CH_2$), 6.5-8.0 ($N^+$-H); Anal. calcd for $C_8H_{18}N_2SBrCl$ $0.75H_2O$: C 31.69, H 6.48, N 9.24, S 10.58, Br 26.36; found: C 31.35, H 6.23, N 9.18, S 10.37, Br 26.51.

(5R,6S) 3[2(1,4-diazabicyclo[2.2.2]octanium-1-yl)ethylthio]-6-(1R-hydroxyethyl-4R-methyl-7-oxo-1-azabicyclo[3.2.0]hept-2-ene-2-carboxylate

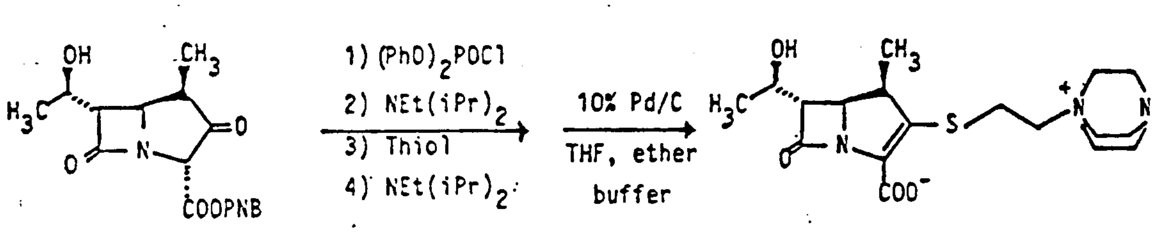


To a cold (5 °C) solution of (5R,6S) paranitrobenzyl 3,7-dioxo-6-(1R-hydroxyethyl)-4R-methyl-1-azabicyclo[3.2.0]heptane-2R-carboxylate (1.0 g, 2.76 mmol) in dry acetonitrile (4 mL) kept under nitrogen atmosphere was added diphenyl chlorophosphate (0.60 mL, 2.88 mmol), dropwise diisopropylethylamine (0.50 mL, 2.88 mol) and 4-dimethylaminopyridine (3 mg). The mature was stirred 5 °C for 0.75 h and treated with a solution of 1-(2-mercaptoethyl)-1,4-diazabicyclo[2.2.2]octanium bromide, hydrochloride (0.956 g, 3.3 mol) in water (4 mL) and diisopropylethylamine (1.06 mL, 6.1 mmol). The mixture was stirred at 5 °C for 2 h, concentrated to about 4 mL and poured on top of the reversed phase column (2.7x11 cm). A mixture of 10-35% acetonitrile in water was used as eluting solvent. Lyophylization of appropriate fractions gave the paranitrobenzyl carbapenem as a mixture of chloride-diphinylphosphate salt, 1.29 g; ir (KBr) $\nu_{max}$: 3700-3100 (OH), 1768 (C=O of β-lactam), 1705 (C=O of ester), 1592, 1520 ($NO_2$), 1345 ($NO_2$) $cm^{-1}$; $^1$Hmr (DMSO, d-6) δ: 1.19 ('d', J=6.4 Hz, $CH_3CHOH$, $CH_3$ on C-4), 5.40 (center of ABq, $J_{ab}$=13.9 Hz, $CH_2$ of PNB ester), 7.73 (d, J=8.6 Hz, Ho of PNB), 8.25 (d, J=8.6 Hz, Hm of PNB). A part of the paranitrobenzyl carbapenem (0.95 g) was solubilized in wet tetrahydrofuran (47 mL) and to the resulting solution was added potassium dihydrogenphosphate-sodium-hydroxide buffer (0.2M, 20 mL), ether (47 mL) and 10% palladium on charcoal (0.95 g). The resulting mixture was hydrogenated under 40 psi for 1 h at 23 °C and filtered on glass fiber filter paper. The two phases were separated; the organic phase was extracted with water (2x10 mL) and the aqueous phases were washed with ether (2x20 ml), pumped under high vacuum and poured on top of the reverse phase column (2.7x16cm). Elution with water gave after lyophilization of appropriate fractions crude material. Repurification on reversed phase gave pure title compound, 0.34 g 24% (for the two steps); ir (KBr) $\nu_{max}$: 3700-3100 (OH), 1750 (C=O of β-lactam), 1595 carboxylate) $cm^{-1}$; $^1$Hmr ($D_2O$) δ: 1.27 (d, J=6.7 Hz, $CH_3$ on C-4), 1.34 (d, J=6.0 Hz, $CH_3CHOH$), 4.2-4.6 (m, $SCH_2CH_2N^+$); uv ($H_2O$) $\lambda_{max}$: (ε9561); $[\alpha]_D^{23}$ +92.4° (c 0.36, $H_2O$); $\tau_{1/2}$ = 16.4 h measured at 36.8 °C in buffer pH 7.4 for a concentration of $10^{-4}$ M.

Example 2

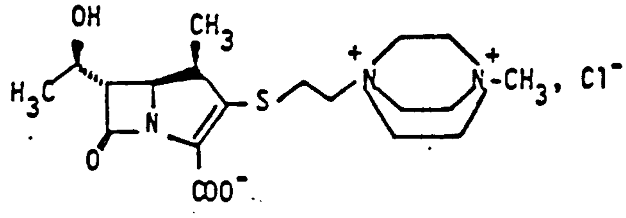

**(5R,6S) 6-(1R-hydroxyethyl)-4R-methyl-3-[2-(4-methyl-1,4-diazabicyclo[2.2.2]octanium-1-yl)ethylthio]-7-oxo-1-azabicyclo[3.2.0]hept-2-ene-2-carboxylate, chloride salt**

1-(2-acetylthioethyl)-4-methyl-1,4-diazabicyclo[2.2.2]octanium bromide, iodide

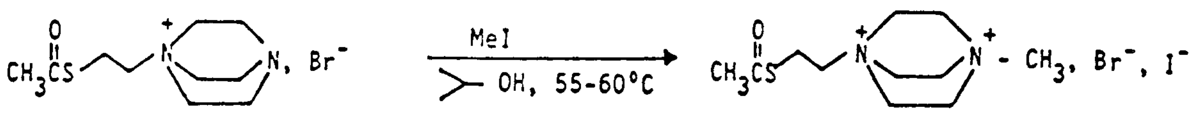


A suspension of 1-(2-acetylthioethyl)-1,4-diazabicyclo [2.2.2) octanium bromide (8.5 g, 28.8 mmol) in isopropyl alcohol (60 mL) was treated with methyl iodide (2.15 mL, 34.5 mmol) and stirred at 55 - 60 ° C for 30 h. After cooling to 23 ° C, the solvent was evaporated leaving a solid which was triturated in hexane and filtered, 12.2 g, 97%. Recrystallization from aqueous ethanol gave an analytical sample, mp 235 ° dec.; Anal. calcd. for $C_{11}H_{22}N_2OSBrI$ 1.25 $H_2O$: C 28.74, H 5.37, N 6.09, S 6.97; found: C 28.42, B 5.15, N 6.09, S 7.03; ir(KBr) $\nu_{max}$: 1695 (C=O) $cm^{-1}$ $^1$Hmr (D$_2$O) $\delta$: 2.51 (s, $CH_3COS$), 3.50 (s, $N^+$-$CH_3$), 3.7-4.1 (m, $CH_3COSCH_2$ $CH_2N^+$), 4.19 (s, $N^+$-$(CH_2CH_2)_3N^+$).

1-(2-mercaptoethyl)-4-methyl-1,4-diazabicyclo[2.2.2]octanium dichloride

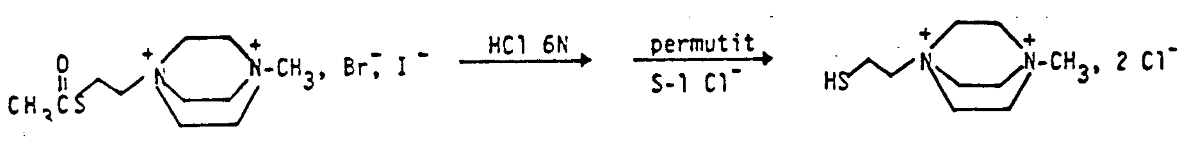


A solution of 1-(2-acetylthioethyl)-4-methyl-1,4-diazabicyclo[2.2.2]octanium bromideiodide (7.0 g, 16.0 mmol) in hydrochloric acid (6N, 60 mL) was stirred under nitrogen atmosphere at 63 ° C for 2.5 h and then concentrated under reduced pressure. The traces of hydrochloric acid were removed by codistillation with water. The resulting syrup was passed through a column of permutit S-1 $Cl^-$ (3 x 36 mL) at a rate of 2.0 mL/min. The aqueous solution was lyophilized leaving a white solid which was dried over $P_2O_5$ under high vacuum for 16 h, 3.45 g, 83.2%; ir(KBr) $\nu_{max}$ :2480 (SH) $cm^{-1}$; $^1$Hmr ($D_2O$)$\delta$: 2.8 - 3.3 (m, $HSCH_2$), 3.46 (s, $N^+$-$CH_3$), 3.6 - 4.0 (m, $SCH_2CH_2$-$N^+$), 4.13 (s, $N^+$-$(CH_2CH_2)_3$-$N^+$);Anal. calcd. for $C_9H_{20}N_2SCl_2$ 1.25 $H_2O$: C 38.37, H 8.05, N 9.94, S 11.38, Cl 25.17; found; C 38.27, H 7.97, N 9.97, S 11.42, Cl 24.94.

(5R,6S) 6-(1R-hydroxyethyl)-4R-methyl-3-[2-(4-methyl-1,4-diazabicyclo[2.2.2]octanium-1-yl)ethylthio]-7-oxo-1-azabicyclo[3.2.0] hept-2-ene-2-carboxylate, chloride salt

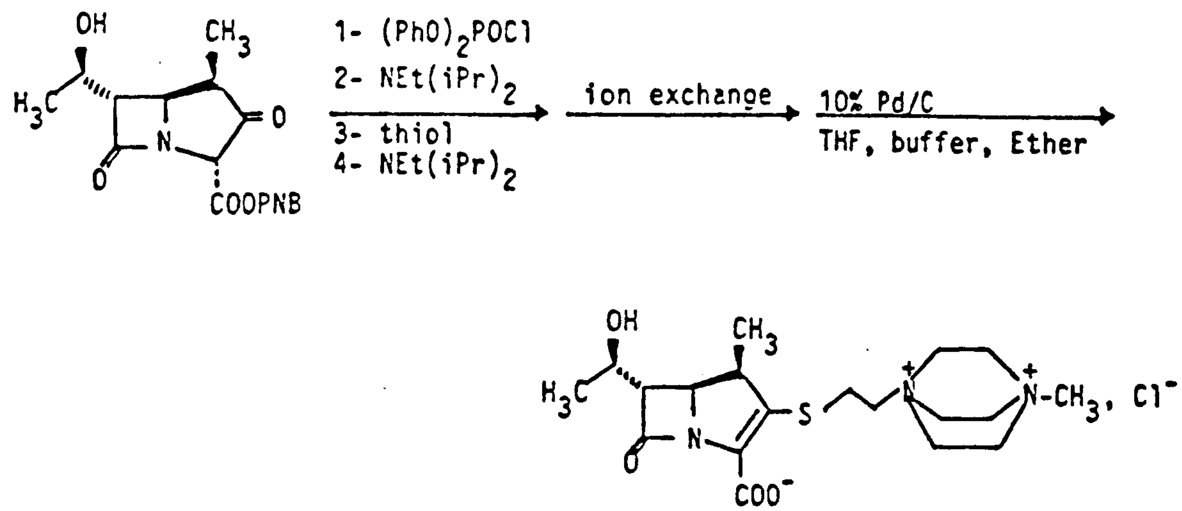


To a cold (5°C) solution of (5R,6S) paranitrobenzyl 3,7-dioxo-6-(1R-hydroxyethyl)-4R-methyl-1-azabicyclo[3.2.0]heptane-2R-carboxylate (1.0 g, 2.76 mmol) in dry acetonitrile (4 mL) kept under nitrogen atmosphere was added diphenyl chlorophosphate (0.60 mL, 2.88 mmol), dropwise diisopropylethylamine (0.50 mL, 2.88 mmol) and 4-dimethylaminopyridine (3 mg). The mixture was stirred at 5°C for 0.75 h before being treated with a solution of 1-(2-mercaptoethyl)-4-methyl-1,4-diazabicyclo[2.2.2]octanium dichloride (0.86 g, 3.3 mmol) in water (3 mL) and diisopropylethylamine (0.58 mL, 3.3 mmol). After 2 h of stirring at 5°C, the mixture was concentrated, treated with some permutit S-1 $Cl^-$ and poured on reverse phase column (2.7 x 11 cm). A mixture of 35 - 45% acetonitrile in water was used as eluting solvent and the fractions containing the carbapenem were passed through a column (1.2 x 50 cm) of pemutit S-1 $Cl^-$. The appropriate fractions were lyophylized to give 0.27 g; ir (KBr)$\nu_{max}$: 3700-3100 (OH), 1765 (C=O of $\beta$-lactam), 1600 (C=O of ester), 1605, 1520 ($NO_2$), 1345 ($NO_2$) $Cm^{-1}$; $^1$Hmr ($D_2O$) $\delta$: 1.32 (d, J = 7.6 Hz, $CH_3$ on C-4), 1.35 (d, J = 6.2 Hz, $CH_3CHOH$), 5.44 (center of ABq, $J_{ab}$ = 14 Hz, $CH_2$ of PNB), 7.67 (d, J = 8.6 Hz, Ho of PNB), 8.26 (d, J = 8.6 Hz, Hm of PNB). A part of the compound obtained previously was solubilized into potassium dihydrogen-sodium hydroxide buffer (0.2 M, 6.2 mL) and to the resulting solution was added ether (16 mL), tetrahydrofuran (8 mL) and 10% Palladium on charcoal (0.25 g). The resulting mixture was hydrogenated under 35 psi at 23°C for 1 h and filtered. The two phases were separated; the organic phase was extracted with cold water (2 x 5 mL) and the aqueous phases were washed with ether (2 x 13 mL), pumped under high vacuum and poured on top of reverse column (3 x 10 cm). Elution with water gave after lyophilization of appropriate fractions a yellow solid, 0.097 g, 9% (for the two steps); ir (KBr)$\nu_{max}$: 3700 - 3100 (OH), 1750 (C=O of $\beta$-lactam), 1592 (carboxylate) $cm^{-1}$; $^1$Hmr ($D_2O$)$\delta$: 1.25 ('d', J = 6.6 Hz, $CH_3$ on C-4), 1.34 ('d', J = 6.1 Hz, $CH_3CHOH$), 3.44 (s, $\overset{+}{N}CH_3$); uv ($H_2O$) $\lambda_{max}$: 296 ($\epsilon$ 7792);

$$[\alpha]_D^{23°C}$$

+ 53.6° (C 0.35, $H_2O$); $T_{1/2}$ = 17.2 h measured at 36.8°C in buffer pH 7.4 for a concentration of $10^{-4}$ M.

## Claims

1. A compound having the formula

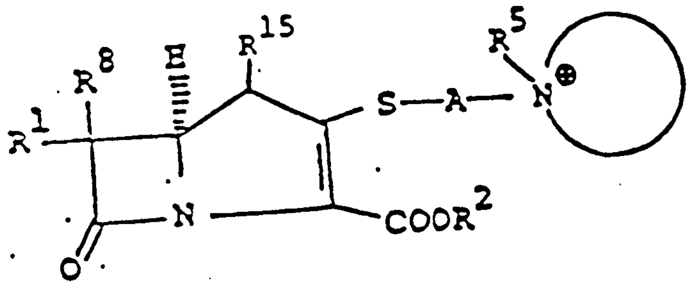

I

wherein

$R^8$ is hydrogen and $R^1$ is selected from the group consisting of hydrogen; substituted and unsubstituted alkyl having from 1 - 10 carbon atoms,
wherein the substituent is selected from the group consisting of

$-OR^3$

$$-O\overset{O}{\overset{\|}{C}}R^3$$

$-OSO_3R^3$

$-OSO_2R^3$

wherein $R^3$ is selected from hydrogen and alkyl having from 1 - 10 carbon atoms;
or
wherein $R^1$ and $R^8$ taken together represent $C_2$-$C_{10}$ alkylidene or $C_2$-$C_{10}$ alkylidene substituted by hydroxy;

$R^{15}$ is selected from the group consisting of alkyl having from 1 - 10 carbon atoms;

A is cyclopentylene, cyclohexylene or $C_2$-$C_6$ alkylene optionally substituted by one or more $C_1$-$C_4$ alkyl groups; $R^2$ is hydrogen, an anionic charge or a conventional readily removable carboxyl protecting group, providing that when $R^2$ is hydrogen or a protecting group, there is also present a counter ion; and

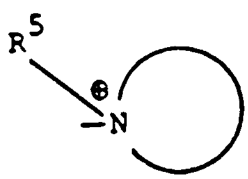

represents

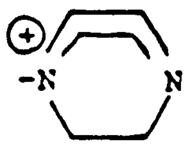

or

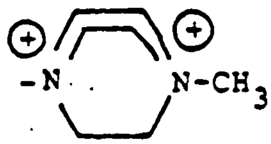

or a pharmaceutically acceptable salt thereof.

**2.** The compounds of claim 1 having the formula

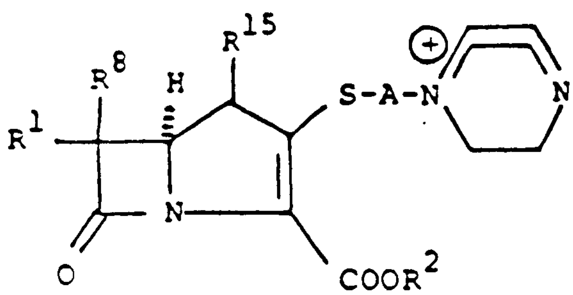

wherein
$R^1$, $R^8$, $R^2$, $R^{15}$ and A are as defined in claim 1.

**3.** The compounds of claim 1 having the formula

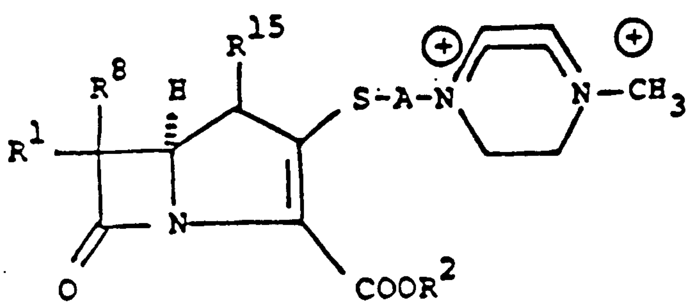

wherein
$R^1$, $R^8$, $R^2$, $R^{15}$ and A are as defined in claim 1.

4. A compound according to anyone of claims 1 to 3 wherein $R^1$ is hydrogen,

$CH_3CH_2$-,

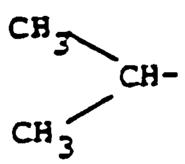 , 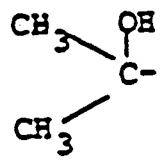 or 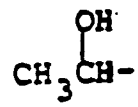

5. A compound according to anyone of claims 1 to 3 wherein $R^1$ and $R^8$ taken together represent

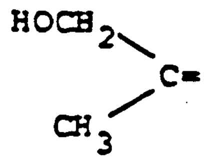

6. A compound according to anyone of claims 1 to 3 wherein $R^1$ is

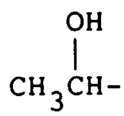

7. A compound according to anyone of claim 1 to 3 wherein $R^1$ is

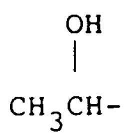

and the absolute configuration is 5R, 6S, 8R.

8. A compound according to anyone of the preceding claims wherein

A is $-CH_2CH_2-$ , $-CH_2CH_2CH_2-$,

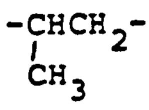 , 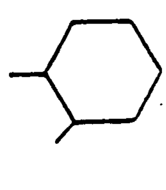 or 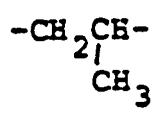 .

9. A compound according to anyone of the preceding claims wherein $R^{15}$ is $\beta$-methyl.

**10.** A compound of the following formulae:

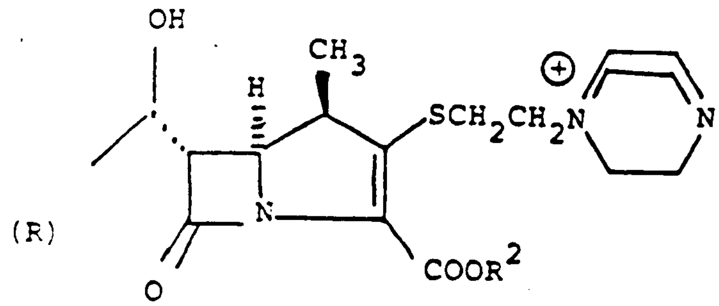

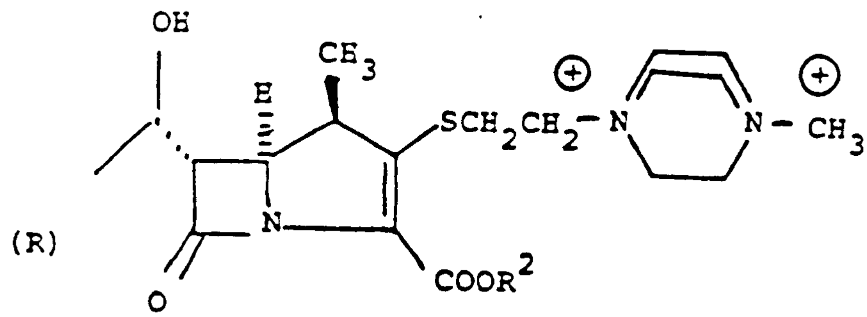

wherein $R^2$ is hydrogen, an anionic charge or a conventional readily removable carboxyl protecting group, providing that when $R^2$ is hydrogen or a protecting group, there is also present a counter ion; or a pharmaceutically acceptable salt thereof.

**11.** The compound according to Claim 10 wherein $R^2$ is p-nitrobenzyl or allyl.

**12.** The compound according to Claim 10 wherein $R^2$ is an anionic charge.

**13.** A process for the preparation of the compounds of claim 1, which process comprises the steps of

(1) reacting an intermediate of the formula

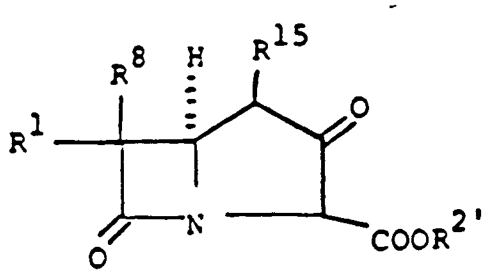

III

wherein $R^1$, $R^{15}$ and $R^8$ are as defined in claim 1 and $R^{2'}$ is a conventional readily removable carboxyl protecting group in an inert organic solvent with diphenyl chlorophosphate in the presence

of base to give an intermediate of the formula

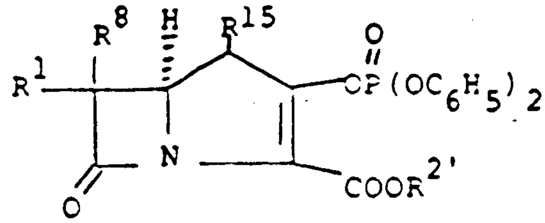

IV

wherein $R^1$, $R^8$, $R^{15}$ and $R^{2'}$ are as defined above;

(2) reacting intermediate IV in an inert organic solvent and in the presence of base with a mercaptan reagent of the formula

HS-A-OH

wherein A is as defined HS-A-OH in claim 1 to give an intermediate of the formula

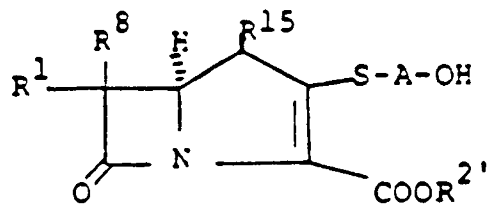

V

wherein $R^1$, $R^8$, $R^{15}$, A and $R^{2'}$ are as defined above;

(3) reacting an intermediate V in an inert organic solvent and in the presence of base with methanesulfonyl chloride or a functional acylating equivalent thereof to give an intermediate of the formula

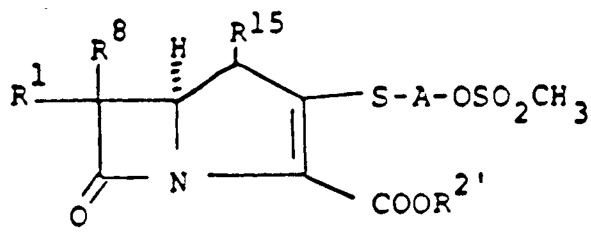

VI

wherein $R^1$, $R^8$, $R^{15}$, A and $R^{2'}$ are as defined above;

(4) reacting intermediate VI in an inert organic solvent with a source of iodide ion so as to displace the methanesulfonyloxy group with an iodo group and form an intermediate of the formula

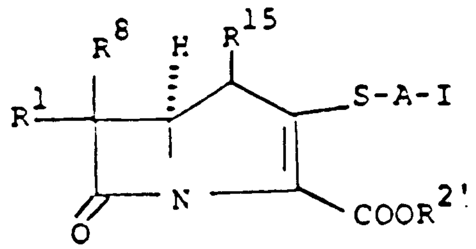

II

wherein $R^1$, $R^8$, $R^{15}$, A and $R^2$ are as defined above; and

(5) subjecting intermediate II to nucleophilic displacement in an inert organic solvent and in the presence of silver ion with an amine of the formula

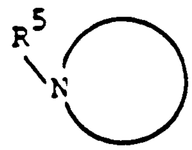

wherein

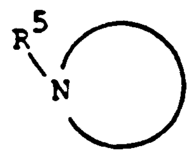

is as defined in claim 1 so as to form a compound of the formula

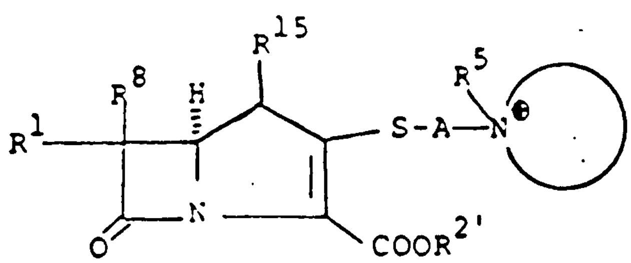

$X^{\ominus}$

wherein $X^{\ominus}$ is a counter ion and $R^1$, $R^8$, $R^{15}$, A, $R^5$,

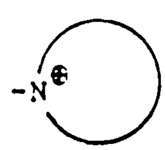

and $R^{2'}$ are as defined above, and, if desired, removing the carboxyl protecting group $R^{2'}$ to give the corresponding de-blocked compound of formula I, and, if desired, converting the so obtained compound into a pharmaceutically acceptable salt thereof.

**14.** A process for the preparation of the compounds of claim 1 which comprises reacting an intermediate of the formula

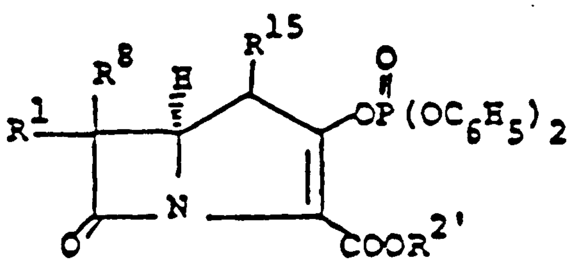

IV

therein $R^1$, $R^{15}$ and $R^8$ are as defined above and $R^{2'}$ is a conventional readily removable carboxyl protecting group with a thiol compound of the formula

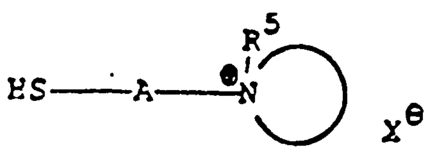

VII

wherein A and

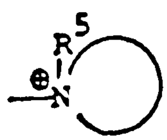

are as defined in claim 1 and $X^{\ominus}$ is a counter anion in an inert solvent and in the presence of base to produce the carbapenem product of the formula

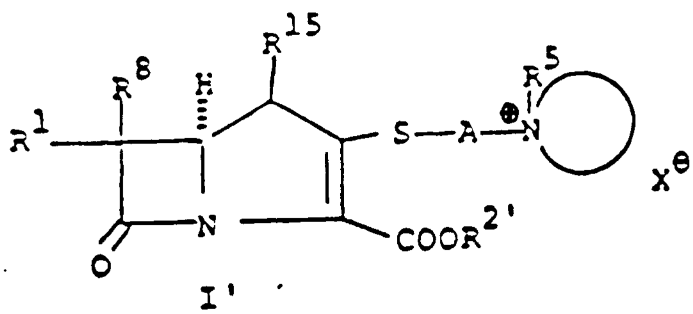

I'

wherein $R^1$, $R^8$, $R^{2'}$, $R^{15}$, A,

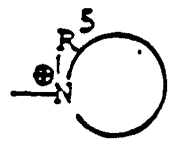

and $X^-$ are as defined above and, if desired removing the carboxyl protecting group $R^{2'}$ to give the corresponding de-blocked compound of Formula I, and, if desired, converting the so obtained compound into a pharmaceutically acceptable salt thereof.

**15.** Pharmaceutical composition comprising at least one compound of anyone of claims 1 to 12, optionally together with common carriers and adjuvants.

**Revendications**

**1.** Composé ayant la formule

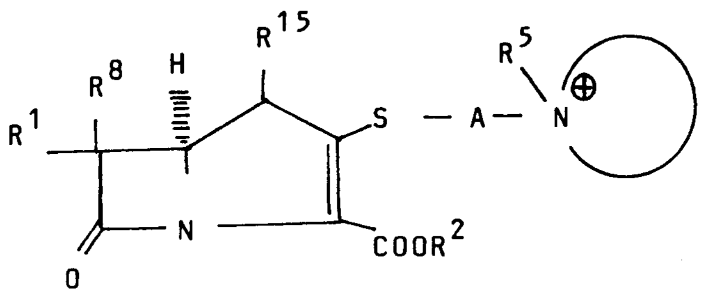

I

où

$R^8$ est hydrogène et $R^1$ est choisi dans le groupe consistant en hydrogène; alkyle substitué et non substitué ayant 1 à 10 atomes de carbone,

où le substituant est choisi dans le groupe consistant en

$-OR^3$

$$-O\overset{O}{\overset{\|}{C}}R^3$$

$-OSO_3R^3$

$-OSO_2R^3$

où $R^3$ est choisi parmi hydrogène et alkyle ayant 1 à 10 atomes de carbone;

ou

où $R^1$ et $R^8$ pris ensemble représentent alkylidène $C_2$-$C_{10}$ ou alkylidène $C_2$-$C_{10}$ substitué par hydroxy;

$R^{15}$ est choisi dans le groupe consistant en alkyle ayant 1 à 10 atomes de carbone; A est cyclopentylène, cyclohexylène ou alkylène $C_2$-$C_6$ facultativement substitué par un groupe alkyle $C_1$-$C_4$ ou plus; $R^2$ est hydrogène, une charge anionique ou un groupe protecteur carboxyle traditionnel facile à enlever, à condition que quand $R^2$ est hydrogène ou un groupe protecteur, il y ait également un contre-ion; et

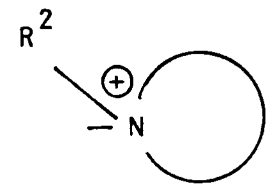

représente

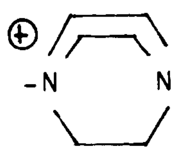

ou

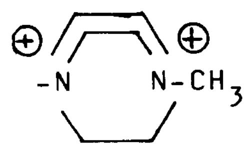

ou un sel acceptable en pharmacie.

**2.** Composés de la revendication 1 ayant pour formule

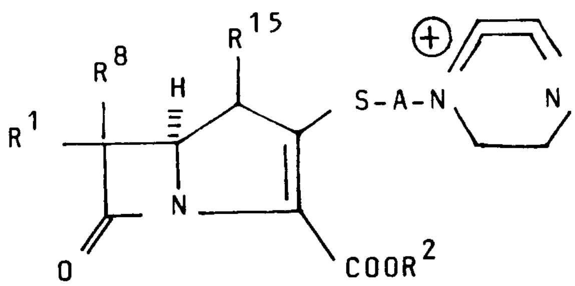

où
$R^1$, $R^8$, $R^2$, $R^{15}$ et A sont tels que définis à la revendication 1.

**3.** Composés de la revendication 1 ayant pour formule

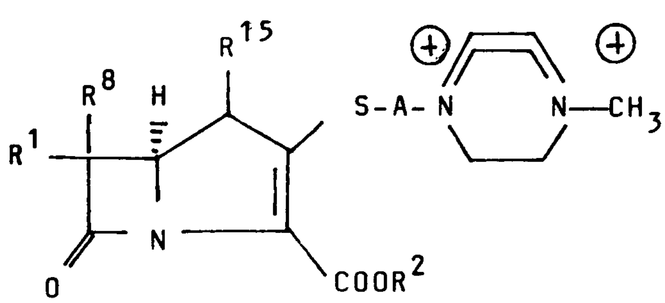

où
$R^1$, $R^8$, $R^2$, $R^{15}$ et A sont tels que définis à la revendication 1.

4. Composé selon l'une quelconque des revendications 1 à 3, où $R^1$ est hydrogène,

$CH_3CH_2$-,

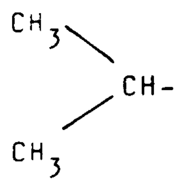

,

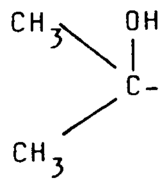

ou

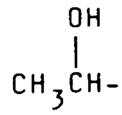

5. Composé selon l'une quelconque des revendications 1 à 3, où $R^1$ et $R^8$ pris ensemble représentent

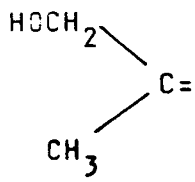

6. Composé selon l'une quelconque des revendications 1 à 3, où $R^1$ est

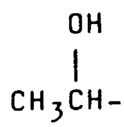

7. Composé selon l'une quelconque des revendications 1 à 3, où $R^1$ est

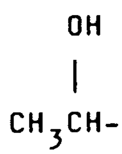

et la configuration absolue est 5R, 6S, 8R.

8. Composé selon l'une quelconque des revendications précédentes, où

A est $-CH_2CH_2-$, $-CH_2CH_2CH_2-$,

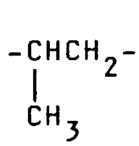

,

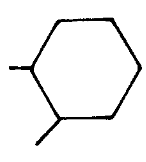

ou

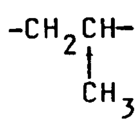

**9.** Composé selon l'une quelconque des revendications précédentes, où $R^{15}$ est $\beta$-méthyle.

**10.** Composé des formules suivantes :

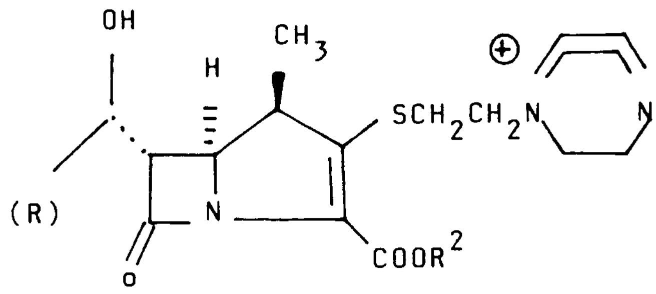

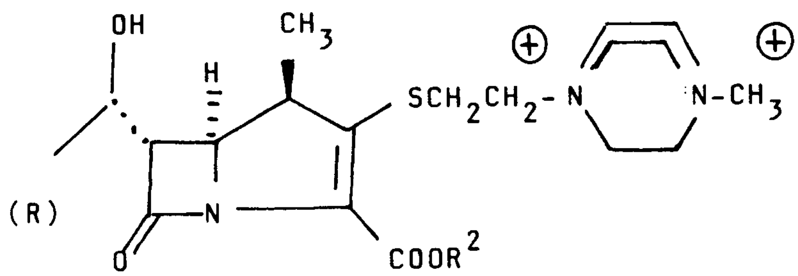

où $R^2$ est hydrogène, une charge anionique ou un groupe carboxyle protecteur conventionnel facile à enlever, à condition que quand $R^2$ est hydrogène ou un groupe protecteur, il y ait également un contre-ion; ou bien un sel acceptable en pharmacie.

**11.** Composé selon la revendication 10, où $R^2$ est p-nitrobenzyle ou allyle.

**12.** Composé selon la revendication 10, où $R^2$ est une charge anionique.

**13.** Procédé pour la préparation des composés de la revendication 1, lequel procédé comprend les étapes de :

(1) faire réagir un intermédiaire de la formule

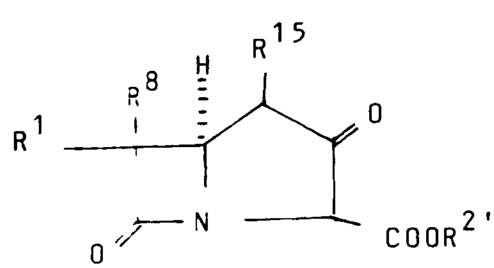

III

où $R^1$, $R^{15}$ et $R^8$ sont tels que définis à la revendication 1 et $R^{2'}$ est un groupe carboxyle protecteur conventionnel facile à enlever dans un solvant organique inerte avec du chlorophosphate

de diphényle en présence d'une base pour donner un intermédiaire de la formule

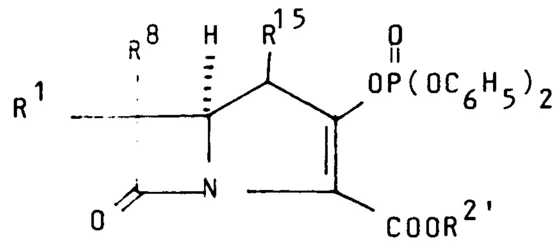

IV

où $R^1$, $R^8$, $R^{15}$ et $R^{2'}$ sont tels que définis ci-dessus;

(2) faire réagir l'intermédiaire IV dans un solvant organique inerte et en présence d'une base avec un mercaptan réactif de la formule

HS-A-OH

où A est tel que défini à la revendication 1 pour donner un intermédiaire de la formule

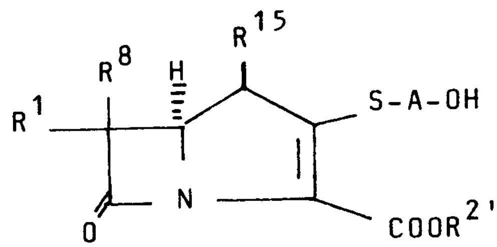

V

où $R^1$, $R^8$, $R^{15}$, A et $R^{2'}$ sont tels que définis ci-dessus;

(3) faire réagir un intermédiaire V dans un solvant organique inerte et en présence d'une base avec du chlorure de méthanesulfonyle ou son équivalent acylant fonctionnel pour donner un intermédiaire de la formule

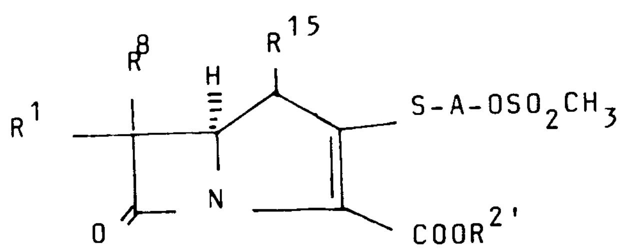

VI

où $R^1$, $R^8$, $R^{15}$, A et $R^{2'}$ sont tels que définis ci-dessus;

(4) faire réagir l'intermédiaire VI dans un solvant organique inerte avec une source de l'ion iodure afin de déplacer le groupe méthanesulfonyloxy avec un groupe iodo et de former un intermédiaire de la formule

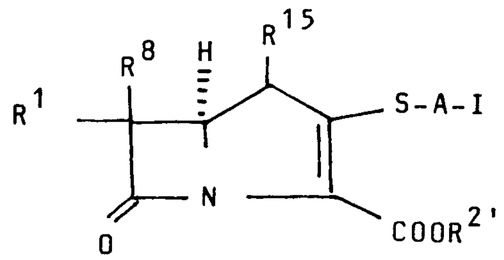

II

où $R^1$, $R^8$, $R^{15}$, A et $R^{2'}$ sont tels que définis ci-dessus; et

(5) soumettre l'intermédiaire II à un déplacement nucléophile dans un solvant organique inerte et en présence de l'ion argent avec une amine de la formule

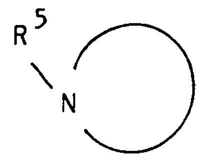

où

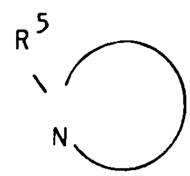

est tel que défini à la revendication 1, afin de former un composé de la formule

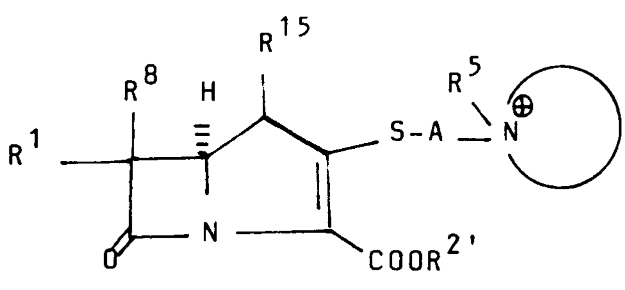

$X^{\ominus}$

où $X^{\ominus}$ est un contre-ion et $R^1$, $R^8$, $R^{15}$, A, $R^5$,

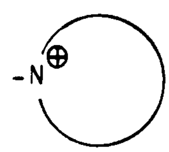

et $R^{2'}$ sont tels que définis ci-dessus et, si on le souhaite, enlever le groupe carboxyle protecteur $R^{2'}$ pour donner le composé débloqué correspondant de formule I, et si on le souhaite, convertir le composé ainsi obtenu en son sel acceptable en pharmacie.

**14.** Procédé pour la préparation des composés de la revendication 1, qui comprend la réaction d'un intermédiaire de formule

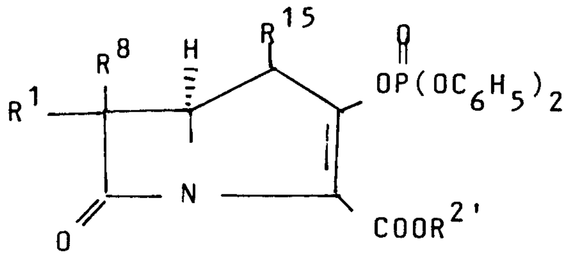

IV

où $R^1$, $R^{15}$ et $R^8$ sont tels que définis ci-dessus et $R^{2'}$ est un groupe carboxyle protecteur conventionnel facile à enlever, avec un composé thiol de formule

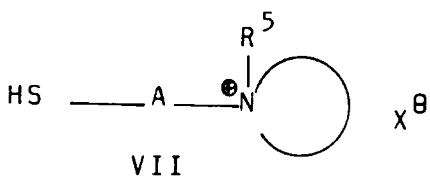

VII

où A et

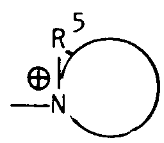

sont tels que définis à la revendication 1 et $X^{\ominus}$ est un contre anion dans un solvant inerte et en présence d'une base pour donner le composé de carbapénème de la formule

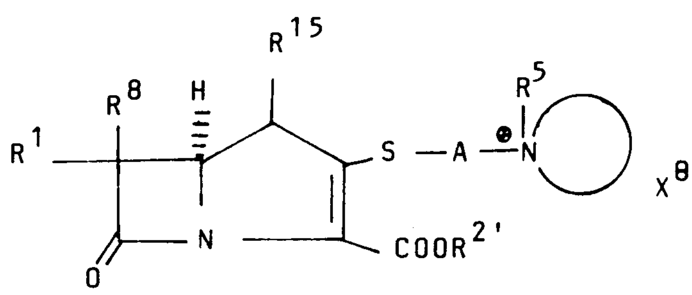

I'

où $R^1$, $R^8$, $R^{2'}$, $R^{15}$, A,

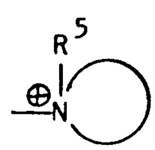

et et $X^-$ sont tels que définis ci-dessus et, si on le souhaite, enlever le groupe carboxyle protecteur $R^{2'}$ pour donner le composé débloqué correspondant de formule I, et si on le souhaite, convertir le composé ainsi obtenu en un sel acceptable en pharmacie.

**15.** Composition pharmaceutique comprenant au moins un composé selon l'une quelconque des revendications 1 à 12, facultativement avec des supports et adjuvants courants.

**Patentansprüche**

**1.** Verbindungen der Formel I

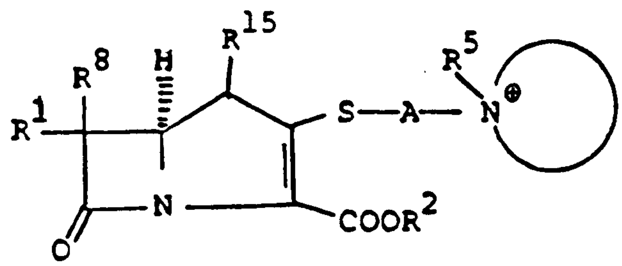

I

worin

$R^8$ für ein Wasserstoffatom steht und $R^1$ ausgewählt ist unter einem Wasserstoffatom, substituiertem und nichtsubstituiertem Alkyl mit 1 - 10 Kohlenstoffatomen, wobei der Substituent ausgewählt ist unter

$-OR^3$

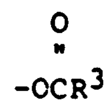

$-OSO_3R^3$

$-OSO_2R^3$

worin $R^3$ ausgewählt ist unter einem Wasserstoffatom und Alkyl mit 1 - 10 Kohlenstoffatomen; oder

worin $R^1$ und $R^8$ zusammen für $C_2$-$C_{10}$-Alkyliden oder für durch Hydroxy substituiertes $C_2$-$C_{10}$-Alkyliden stehen;

$R^{15}$ ausgewählt ist unter Alkyl mit 1 - 10 Kohlenstoffatomen;

A für cyclopentylen, Cyclohexylen oder $C_2$-$C_6$-Alkylen, das gegebenenfalls mit einer oder mehreren $C_1$-$C_4$-Alkylgruppen substituiert ist, steht; $R^2$ für ein Wasserstoffatom, eine anionische Ladung oder eine herkömmliche leicht entfernbare Carboxylschutzgruppe steht, vorausgesetzt, daß auch ein Gegenion vorhanden ist, wenn $R^2$ für ein Wasserstoffatom oder eine Schutzgruppe steht; und

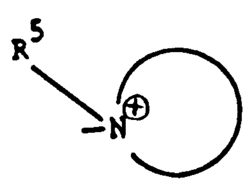

für

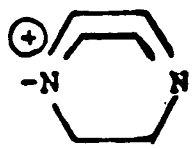 oder 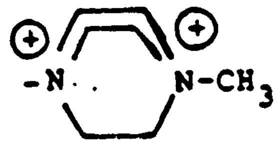

steht,
oder ein pharmazeutisch akzeptables Salz davon.

2. Verbindungen nach Anspruch 1 der Formel

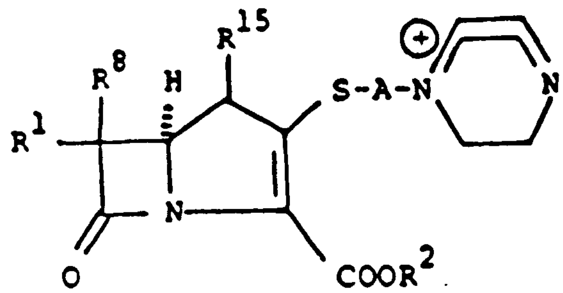

worin $R^1$, $R^8$, $R^2$, $R^{15}$ und A wie in Anspruch 1 definiert sind.

3. Verbindungen nach Anspruch 1 der Formel

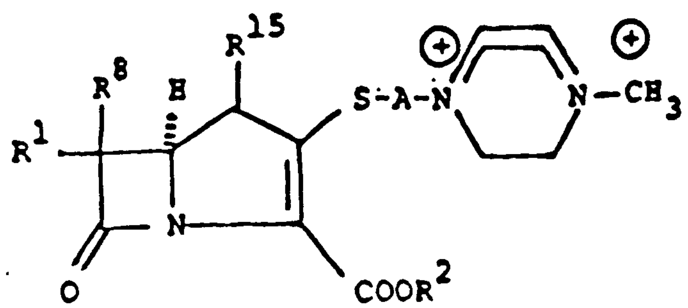

worin $R^1$, $R^8$, $R^2$, $R^{15}$ und A wie in Anspruch 1 definiert sind.

4. Verbindungen nach einem der Ansprüche 1 bis 3, worin $R^1$ für ein Wasserstoffatom, $CH_3CH_2$-,

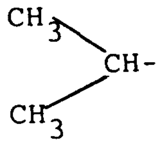 , 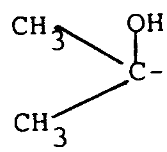 oder 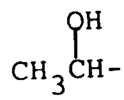

steht.

5. Verbindungen nach einem der Ansprüche 1 bis 3, worin $R^1$ und $R^8$ zusammen für

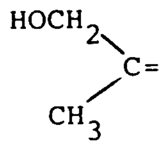

stehen.

6. Verbindungen nach einem der Ansprüche 1 bis 3, worin $R^1$ für

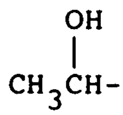

steht.

7. Verbindungen nach einem der Ansprüche 1 bis 3, worin $R^1$ für

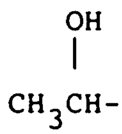

steht und die absolute Konfiguration 5R, 6S, 8R ist.

8. Verbindungen nach einem der vorhergehenden Ansprüche, worin A für $-CH_2CH_2-$, $-CH_2CH_2CH_2-$,

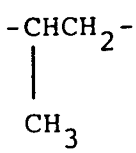 , 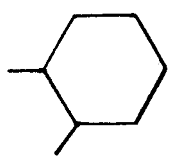 oder 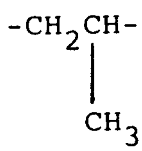

steht.

9. Verbindungen nach einem der vorhergehenden Ansprüche, worin $R^{15}$ für ß-Methyl steht.

**10.** Verbindungen der folgenden Formeln:

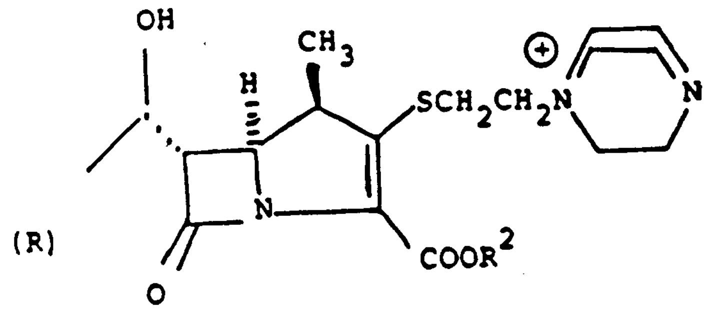

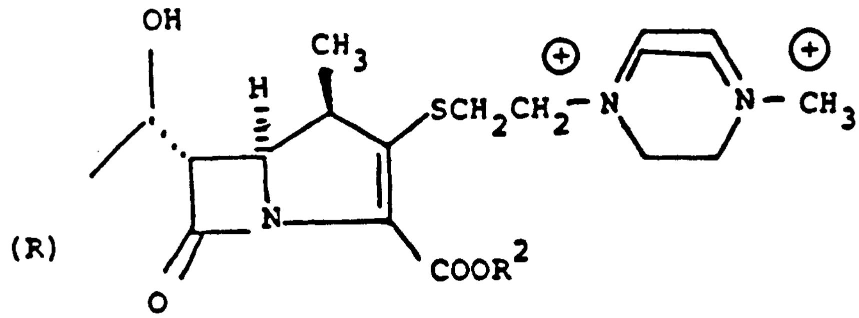

worin $R^2$ für ein Wasserstoffatom, eine anionische Ladung oder eine herkömmliche, leicht entfernbare Carboxylschutzgruppe steht, vorausgesetzt, daß auch ein Gegenion vorhanden ist, wenn $R^2$ für ein Wasserstoffatom oder eine Schutzgruppe steht; oder ein pharmazeutisch akzeptables Salz davon.

**11.** Verbindungen nach Anspruch 10, worin $R^2$ für p-Nitrobenzyl oder Allyl steht.

**12.** Verbindung nach Anspruch 10, worin $R^2$ für eine anionische Ladung steht.

**13.** Verfahren zur Herstellung der Verbindungen nach Anspruch 1, wobei man
(1) ein Zwischenprodukt der Formel III

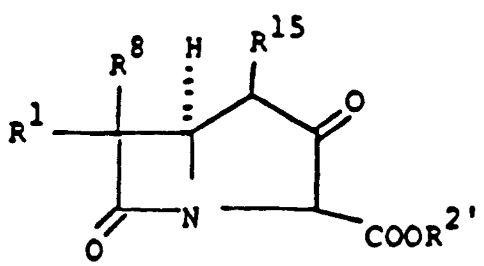

III

worin $R^1$, $R^{15}$, und $R^8$ wie in Anspruch 1 definiert sind und $R^{2'}$ eine herkömmliche leicht entfernbare Carboxylschutzgruppe ist, in einem inerten organische Lösungsmittel mit Diphenylchlorphosphat in

Gegenwart von Base umsetzt, wobei man ein Zwischenprodukt der Formel

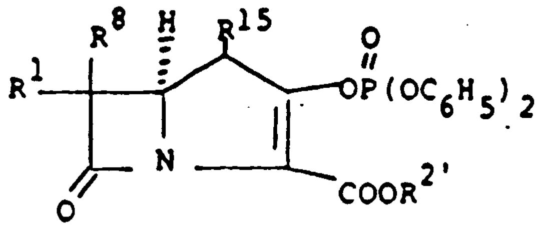

IV

worin $R^1$, $R^8$, $R^{15}$ und $R^{2'}$ wie oben definiert sind, erhält;
(2) das Zwischenprodukt IV in einem inerten organischen Lösungsmittel und in Gegenwart einer Base mit einem Mercaptan-Reagens der Formel

HS-A-OH

worin A wie in Anspruch 1 definiert ist, unsetzt, wobei man ein Zwischenprodukt der Formel

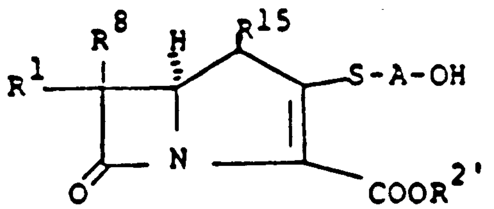

V

worin $R^1$, $R^8$, $R^{15}$, A und $R^{2'}$ wie oben definiert sind, erhält;
(3) ein Zwischenprodukt V in einem inerten organischen Lösungsmittel und in Gegenwart einer Base mit Methansulfonylchlorid oder einem funktionellen acylierenden Äquivalent davon umsetzt, wobei man ein Zwischenprodukt der Formel

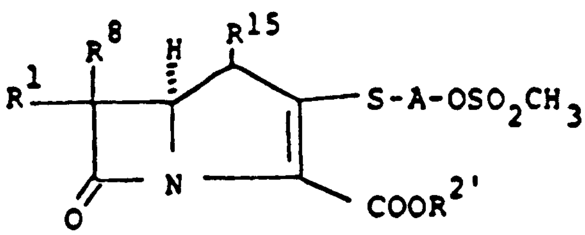

VI

worin $R^1$, $R^8$, $R^{15}$, A und $R^{2'}$ wie oben definiert sind, erhält;

(4) ein Zwischenprodukt VI in einem inerten organischen Lösungsmittel mit einer Jodidionenquelle umsetzt, um die Methansulfonylgruppe mit einer Jodgruppe zu ersetzen wird und ein Zwischenprodukt der Formel

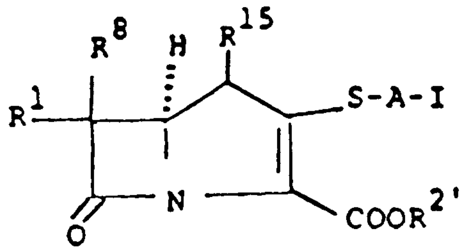

II

worin $R^1$, $R^8$, $R^{15}$, A und $R^{2'}$ wie oben definiert sind, zu bilden; und

(5) das Zwischenprodukt II einer nucleophilen Austauschreaktion in einem inerten organischen Lösungsmittel und in Gegenwart von Silberionen mit einem Amin der Formel

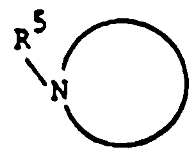

worin

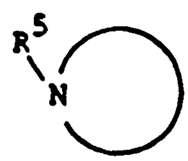

wie in Anspruch 1 definiert ist, unterwirft, wobei man eine Verbindung der Formel

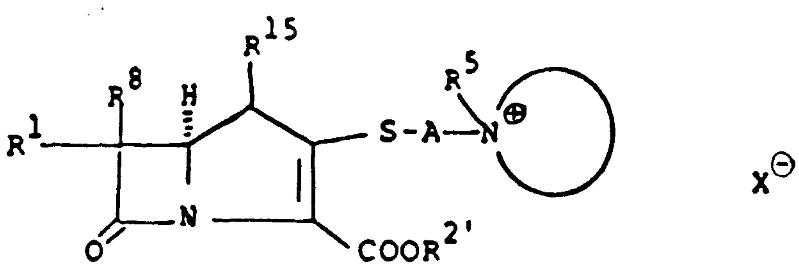

worin $X^{\ominus}$ ein Gegenion ist und $R^1$, $R^8$, $R^{15}$, A, $R^5$,

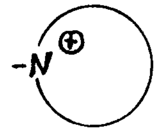

und $R^{2'}$ wie oben definiert sind, erhält und, falls gewünscht, die Carboxylschutzgruppe $R^{2'}$ entfernt, wobei man die entsprechende entblockierte Verbindung der Formel I erhält, und falls gewünscht, die so erhaltene Verbindung in ein pharmazeutisch akzeptables Salz davon überführt.

**14.** Verfahren zur Herstellung der Verbindungen nach Anspruch 1, wobei man ein Zwischenprodukt der Formel

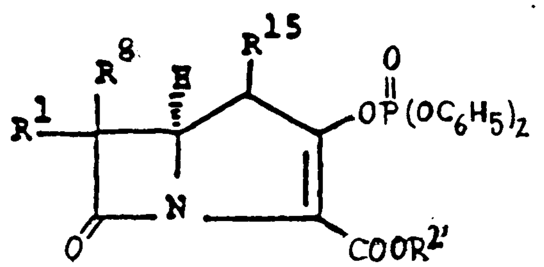

IV

worin $R^1$, $R^{15}$ und $R^8$ wie oben definiert sind und $R^{2'}$ eine herkömmliche leicht entfernbare Carboxylschutzgruppe bedeutet mit einer Thiol-Verbindung der Formel

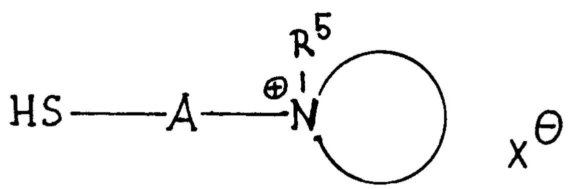

VII

worin A und

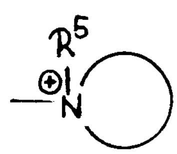

wie in Anspruch 1 definiert sind und $X^{\ominus}$ ein Gegenanion ist, in einem inerten Lösungsmittel und in Gegenwart einer Base umsetzt, wobei man das Carbapenemprodukt der Formel

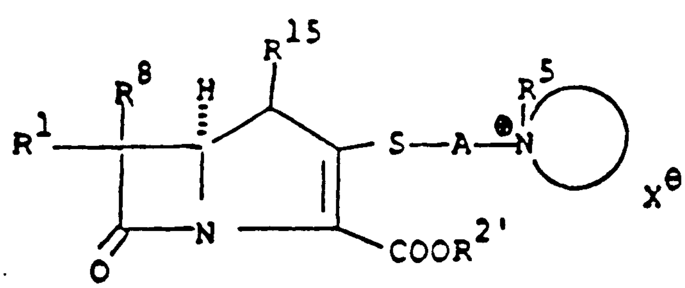

I'

worin $R^1$, $R^8$, $R^{2'}$ $R^{15}$, A,

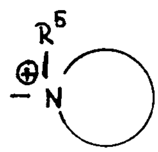

und $X^{\ominus}$ wie oben definiert sind, erhält, und falls gewünscht, die Carboxylschutzgruppe $R^{2'}$ entfernt, wobei man die entsprechende entblockierte Verbindung der Formel I erhält, und falls gewünscht, die so erhaltenen Verbindung in ein pharmazeutisch akzeptables Salz davon umsetzt.

**15.** Pharmazeutisches Mittel, umfassend mindestens eine Verbindung der Ansprüche 1 bis 12, gegebenenfalls zusammen mit herkömmlichen Trägern und Adjuvantien.